# EUROPEAN PATENT SPECIFICATION

(11) **EP 3 466 387 B1**
(45) Date of publication and mention of the grant of the patent: **27.09.2023**
(21) Application number: 17195498.5
(22) Date of filing: 09.10.2017
(51) Int. Cl.: A61F 13/534, A61F 13/15

(54) **METHOD AND APPARATUS FOR MANUFACTURING AN ABSORBENT STRUCTURE**
VERFAHREN UND VORRICHTUNG ZUR HERSTELLUNG EINER ABSORPTIONSSTRUKTUR
PROCÉDÉ ET APPAREIL DE FABRICATION D'UNE STRUCTURE ABSORBANTE

(43) Date of publication of application: 10.04.2019
(73) Proprietor: Drylock Technologies NV, 9240 Zele (BE)
(72) Inventor: Van Ingelgem, Werner, 9240 Zele (BE); Smet, Steven, 9240 Zele (BE); Derycke, Tom, 9240 Zele (BE); Verduyn, Dries, 9240 Zele (BE)
(74) Representative: D'Halleweyn, Nele Veerle Trees Gertrudis

(56) References cited:
- WO-A1-2016/108039
- GB-A- 2 263 914
- JP-A- 2002 159 533

## Description

### TECHNICAL FIELD

The present invention pertains to the technical field of absorbent articles, and relates to a method and apparatus for manufacturing such an absorbent article and/or absorbent structure.

### BACKGROUND

Absorbent articles such as diapers, baby pants, adult incontinent garments and the like, typically comprise an absorbent core, positioned in between a liquid permeable or pervious, hydrophilic or semi hydrophilic topsheet and a liquid impermeable or impervious backsheet. The absorbent core comprises absorbent material that is able to absorb fluid and liquid bodily excretions of the user of the absorbent article.

The absorbent material of the absorbent core may be an absorbent particulate polymer material which is dispersed in a matrix of cellulose fibers or fluff pulp in order to prevent the particulate material from aggregating, as well as to prevent gel blocking. Gel blocking can occur when the absorbent particulate polymer material absorbs liquid, as they tend to typically swell and form a gel structure. This gel structure often blocks the further transfer of liquid into the remaining absorbent core. As a result, the liquid may be unable to reach the remaining absorbent particulate polymer material and the efficiency of the overall absorbent article decreases significantly. Existing fluff pulp materials are not suited to cope with rapid, subsequent insults of fluid since they possess limited distribution capacities. Moreover existing fluff pulp materials exhibit a limited capacity of overall liquid intake. Furthermore, existing absorbent cores containing fluff pulp have a limited wet integrity, which leads to the shape and fit of the absorbent article being deformed when e.g. an absorbent article is being worn by a baby which moves around.

In recent years, there has been a strong demand for more flexible, thinner, light-weight, absorbent articles to resolve various problems associated with manufacturing, marketing, design, fit, wearing comfort, distribution, garbage disposal, material and energy consumption, transport and storage costs and the like. This lead to the search for and the development and production of absorbent articles of which the absorbent cores contains little to no cellulose fibers or fluff pulp, as the latter tend to be quite bulky, thus rendering generally more thick absorbent cores which reduces the overall wearing comfort of the user of the absorbent article.

Hence, various absorbent cores containing little to no cellulose fibers or fluff pulp were developed in the past few years to try and overcome the above drawbacks, whereby the relative high amounts of absorbent polymer materials necessary to replace the absorption, distribution and retention capacity of the excluded cellulose fibers and/or fluff pulp were loaded, distributed and immobilized within these new absorbent cores according to several techniques.

One of the major difficulties in producing absorbent structures without fluff is that of combining together the integrity and the absorption capacity of the liquid of the structure. An absorbent core without fluff should have very high values of integrity and of absorption capacity. In reality, what happens is that if priority is given to one of the two characteristics, the other consequently results as being penalized, or rather a high absorption capacity is achieved at the price of an insufficient integrity of the structure and vice versa.

Methods and apparatus have already been proposed that have the object of penetrating granular material within a layer of highly voluminous fibers, in order to produce an absorbent structure without fluff. For example, WO2013/153235 describes a method for producing an absorbent structure comprising at least one fibrous non-woven layer having an empty volume configured to be penetrated by superabsorbent particles. The superabsorbent particles are dispersed into the fibrous layer by means of vacuum and vibrations.

The technology which provides the penetration of superabsorbent particles into a fibrous layer by means of vacuum and vibrations is not sufficiently effective because, in practice, it does not allow a deep penetration of the superabsorbent material granules into the fibrous layer and, consequently, does not allow the distribution of high amounts of superabsorbent material into the fibrous layer.

EP-A-1526214 describes an electrostatic method for the penetration of superabsorbent powder within a fibrous layer. The powder and the fibrous layer are placed between electrodes, which are connected to the poles of an alternating current generator. The electrodes form an electrostatic field with a voltage gradient in the order of 0.10 - 20 kV/mm.

In practice, the electrostatic penetration technology provides better results than the penetration technology with vacuum and vibrations. However, the electrostatic technology is affected by various drawbacks, including the high length of the electrostatic tunnel (in the order of 10 meters), the high cost and safety issues related to the risk of subjecting inflammable material to a high electrical voltage (up to 20 KV).

EP-A-0540041 describes a method for forming a super-absorbent composite material, which comprises a step of hydraulic needling of a sheet of non-woven fabric to increase its distribution properties of the liquids, and to introduce dry superabsorbent material in intimate contact with at least one surface of the hydraulically-needled fibrous sheet. This document describes that the composite sheet may be mechanically softened by calendering, opening, embossing, differential ironing, etc.

EP3153141 A1 discloses an apparatus for producing an absorbent structure without fluff, composed almost exclusively of fibrous material and superabsorbent granular material, wherein he apparatus comprises a volumizing device arranged downstream of an absorbent particles dispensing device. This volumizing device comprises a cylinder having a toothed portion equipped with protruding elements and is configured to penetrate with the protruding elements of the toothed portion the fibrous material to increase the specific volume of the fibrous material. In other words, the volumizing device provided a combing action on the fibrous material. However, this combing action may damage and/or destroy the fibers of the fibrous material, which will cause the dry and wet integrity of the final fluffless absorbent article to deteriorate.

GB 2 263 914 A discloses an absorbent fibrous structure composed of shrinkable bicomponent fibres into which superabsorbent material can be retained. The superabsorbent particles are retained in the fiber structure by heat-treating the structure so as to spiralize the fibres which results in the superabsorbent material being locked mechanically in the windings or turnings of the spiralized bicomponent fibers. Because the bicomponent fibers are melted partially during heat-treatment, they will function as a binder for the fiber structure itself.

WO 2016/108039 A1 discloses a method for dissipating and entrapping SAP particles within air-permeable non-woven structures. SAP is dispersed onto the surface of a nonwoven fabric, and subjected to an external heating process which entraps the SAP particles. More in particular, heat is provided to melt the uppermost layer of the fibrous structure as to create a physical barrier which entraps the SAP particles in the non-woven structure.

JP 2002 159533 A discloses an absorber having SAP held between constituting fibres of a nonwoven fabric. The nonwoven fabric comprises thermally fusible fibers and non-thermally fusible fibers. A carded web is formed by combining different fibers, and this web is treated with hot air to form an air-through nonwoven fabric.

### SUMMARY

The object of embodiments of the invention is to provide an apparatus and a method for manufacturing an absorbent structure which is substantially free of fluff and exhibits a high integrity in both a dry state and a wet state in combination with a high capacity for absorbing fluids. Moreover it is an aim of embodiments of the invention to provide an apparatus and a method for manufacturing an absorbent structure which is substantially free of fluff which apparatus and method overcome the above described problems of prior art solutions.

According to a first aspect of the invention there is provided a method of manufacturing an absorbent structure comprising a substrate layer and superabsorbent particles, said method comprising the steps of:
- providing an essentially endless substrate layer of fibrous non-woven material which is heat sensitive;
- applying heat to said substrate layer; and
- depositing superabsorbent particles on said substrate layer.

Embodiments are based inter alia on the inventive insight that by applying heat to the heat sensitive substrate layer, fibres within the substrate layer will curl under the influence of the heat which causes the substrate layer to open up. This way, an available volume for accommodating superabsorbent particles within the substrate layer is increased, without negatively affecting the wet and/or dry integrity of the absorbent structure. Furthermore, by opening up the fibrous substrate layer by applying heat, a deep penetration of superabsorbent particles within the substrate layer is achieved along with an increased density of superabsorbent particles being distributed within the substrate layer. Although the step of applying heat to the substrate layer is described before the step of depositing superabsorbent particles on the substrate layer, it should be noted that these steps can be executed in either order. In other words, in an embodiment heat is applied to the substrate layer before superabsorbent particles are deposited thereon, and in an alternative embodiment heat is applied to the substrate layer after superabsorbent particles have been deposited thereon. In yet another embodiment, heat may be applied both before and after superabsorbent particles have been deposited on the substrate layer. Also when the superabsorbent particles have been deposited on the fibrous layer prior to the application of heat, the fibrous material will open up thereby increasing a specific volume which is available for the superabsorbent particles which are present in the substrate layer, and the superabsorbent particles will be able to penetrate deeper into the fibrous substrate layer, thereby providing a better distribution of superabsorbent particles within the substrate layer.

As compared with methods known in the art which use mechanical means to increase an available volume for accommodating superabsorbent particles within the substrate layer, applying heat to the substrate layer enables the substrate layer to keep its integrity. Prior art methods typically handle the fibres in the substrate layer mechanically in such a manner that it causes the fibres to be ripped apart, to be detached from the substrate layer, and/or causes upper layers to be detached from under layers etc. This is caused by mechanically treating or combing the fibres either too hard or too long, or a combination of both. In other words, the mechanical treatment of the fibres of the substrate layer weakens the integrity of the substrate layer in wet and/or dry state. This has a negative effect on the strength of the substrate layer and of the absorbent article wherein the substrate layer is ultimately positioned. In situations such as swelling superabsorbent particles when liquid is absorbed, a baby moving while wearing a wetted diaper, a baby lying sideways while wearing a diaper etc. sagging and bunching of the diaper is promoted when the substrate layer, and thereby the absorbent article, does not exhibit a sufficiently high wet integrity or strength. This may cause leakage of the absorbent article and/or failure of the absorbent article. However, by applying heat to the substrate layer in stead of providing a mechanical treatment, the wet and dry integrity of the fibres and the substrate layer are maintained, resulting in a more reliable absorbent article.

Preferably, the heat sensitive non-woven material of the substrate layer is particularly suited to have its specific volume increased by application of heat. In other words, preferably the fibrous non-woven material is selected in such a way that it has a high relofting capability such that upon being heated, fibres of the material can easily open up and create additional volume, space and/or cavities in which superabsorbent particles can be deposited. When the material then cools down, the fibres and/or cavities created therein will close at least partially such that superabsorbent particles are retained within the cavities in between the fibres of the substrate layer. The relofting capability of a material is dependent on at least one of a chemical composition of the material, a spatial shape (of the fibres) of the material, a curvature (of the fibres) of the material, type of fibres, composition of fibres, etc.

According to an embodiment applying heat to the substrate layer is done by directing hot air towards the substrate layer, while the substrate layer is guided along a rotating member. In this manner the substrate layer, which is typically travelling at high speed along the rotating member can efficiently be heated by directing hot air towards the substrate layer.

According to an embodiment the hot air has a temperature between 60°C and 200°C, preferably between 80°C and 180°C, more preferably between 100°C and 160°C and most preferably between 120°C and 150°C. The skilled person understands that a preferred temperature may depend on any one of a material of the substrate layer, a mass of the substrate layer, a gsm of the substrate layer, a thickness of the substrate layer, a speed of moving or guiding the substrate layer, a distance to be travelled by the hot air before reaching the substrate layer, etc.

According to an embodiment the hot air is directed towards the substrate layer from a distance of 1 to 20 cm, preferably 2 to 15cm, more preferably 3 to 12 cm, and most preferably 5 to 10cm from the substrate layer.

According to an embodiment the substrate layer is guided along the rotating member at a speed of at least 1 m/s, preferably at least 2 m/s, more preferably at least 3 m/s, and most preferably at least 3,5 m/s.

According to an embodiment the hot air is directed towards a surface of the substrate layer having a width of at least 50%, preferably at least 60%, more preferably at least 70%, even more preferably 80% and most preferably at least 90% of the width of the substrate layer. In this manner, it can be assured that a sufficiently large surface on which superabsorbent particles will be deposited is properly heated. The width of the substrate layer depends on the intended use of the substrate layer. In other words, substrate layers to be used in baby diapers may have a smaller width as compared to substrate layers to be used in adult incontinence article. Typically a width of a substrate layer for a baby diaper may be between 5 and 15 cm, depending on the size of the baby diaper, whereas a width of a substrate layer for an adult incontinence article may be comprised between 8 and 22cm, approximately. A preferred length of the surface of the substrate layer to which hot air is directed depends on the speed of the substrate layer, thickness of the substrate layer, material of the substrate layer and distance to be travelled by the hot air before reaching the substrate layer. Exemplary lengths may be between 1cm and 50cm, preferably between 5cm and 40 cm, and more preferably between 10cm and 30cm. It is clear to the skilled person that the substrate layer is moving at a certain speed and that eventually approximately an entire surface of the substrate layer will be affected by the hot air.

According to an embodiment applying heat to the substrate layer is done by guiding the substrate layer through a heating chamber. In this manner the substrate layer, which is typically travelling at high speed along the rotating member can efficiently be heated during the passage of the substrate layer through the high temperature heating chamber. In the heating chamber the substrate layer can take up heat by passing through heated stationary air. Alternatively or in addition, hot air may be directed to the substrate layer in the heating chamber.

According to an embodiment the temperature in the heating chamber is between 60°C and 160°C, preferably between 70°C and 140°C, more preferably between 80°C and 120°C, and most preferably between 90°C and 110°C. The skilled person understands that a preferred temperature may depend on any one of a material of the substrate layer, a mass of the substrate layer, a gsm of the substrate layer, a thickness of the substrate layer, a speed of moving or guiding the substrate layer, etc.

According to an embodiment the substrate layer is guided through the heating chamber at a speed of at least 1 m/s, preferably at least 2 m/s, more preferably at least 3 m/s, and most preferably at least 3,5 m/s.

According to an embodiment the substrate layer is guided through the heating chamber for a distance of 1 to 30m, preferably 2 to 25m, more preferably 3 to 20m, and more preferably 5 to 15m.In an embodiment, the substrate layer is guided along a straight guiding member with a length corresponding to the distance which is travelled by the substrate layer within the heating chamber. In a preferred embodiment, the substrate layer is guided along a guiding member which comprises a plurality of spools or reels such that a path of the substrate layer is divided into a plurality of subpaths which are positioned above each other and/or next to each other. For example, when a substrate layer is guided through the heating chamber for a distance of 15m, this can be achieved by guiding the substrate layer along a straight path with a length of 15m, or this can be achieved by guiding the substrate layer along five subpaths with a length of 3m, ten subpaths with a length of 1,5m, or fifteen subpaths with a length of 1m, wherein the subpaths are mutually connected via spools or reels. It is clear to the skilled person that for a given distance, numerous options of dividing the path along which the substrate layer is guided are possible. By guiding the substrate layer along a plurality of adjacent subpaths which are positioned above, below or next to each other required machine space can be reduced along with costs and energy consumption.

According to an embodiment the method further comprises the step of applying heat to said substrate layer after superabsorbent particles have been deposited thereon. In this manner, superabsorbent particles which are present on and/or within the substrate layer can penetrate deeper into the substrate layer and be distributed more evenly.

According to an embodiment the method further comprises the step of combing the substrate layer before superabsorbent particles are deposited thereon. In addition to applying heat to the substrate layer, combing the substrate layer may lead to a further increase of available specific volume within the substrate layer which is available to accommodate the superabsorbent particles. In this manner, superabsorbent particles which are to be deposited on and/or within the substrate layer can penetrate deeper into the substrate layer and be distributed more evenly. By performing the combing action in combination with applying heat to the substrate layer, the combing may be controlled to have a smaller impact on the integrity and strength of the fibres and/or the substrate layer. In other words, by applying heat to the substrate layer and additional step of combing can be performed which is less aggressive or severe as compared to mechanical treatments in the prior art. Because heat is applied to the substrate the combing can be performed in a manner wherein the fibres are not damaged but a specific volume within the substrate layer is still increased. This can be achieved by either combing the fibres of the substrate layer less deep or with a smaller relative speed as compared to prior art methods.

According to a further embodiment the method further comprises the step of combing the substrate layer after superabsorbent particles have been deposited thereon. In addition to applying heat to the substrate layer, combing the substrate layer may lead to a further increase of available specific volume within the substrate layer which is available to accommodate the superabsorbent particles. In this manner, superabsorbent particles which are present on and/or within the substrate layer can penetrate deeper into the substrate layer and be distributed more evenly.

According to a preferred embodiment the method further comprises the steps of:
- providing an essentially endless top layer;
- applying the top layer on top of the substrate layer; and
- attaching said substrate layer to said top layer, thereby enclosing the superabsorbent particles therebetween.

In this manner an absorbent sandwich structure is created wherein the superabsorbent particles are embedded within the fibers of the substrate layer and wherein the superabsorbent particles are positioned between a bottom surface of the substrate layer and the top layer, such that superabsorbent particles cannot emigrate from the absorbent sandwich structure.

According to an exemplary embodiment the method further comprises the step of sucking the superabsorbent particles into the substrate layer after depositing the superabsorbent particles. In this manner, superabsorbent particles can penetrate even more deeply within the fibres of the substrate layer. The step of sucking the superabsorbent particles can be done by creating a controlled vacuum below the bottom surface of the substrate layer, such that superabsorbent particles are sucked towards the bottom surface of the substrate layer.

According to an embodiment the method further comprises the step of providing an adhesive to the substrate layer.

According to a preferred embodiment providing an adhesive to the substrate layer comprises spraying glue on the substrate layer before and/or after the step of depositing superabsorbent particles on said substrate layer.

The skilled person will understand that the hereinabove described technical considerations and advantages for method embodiments also apply to the below described apparatus embodiments, mutatis mutandis.

According to a second aspect of the invention there is provided an apparatus for manufacturing an absorbent structure comprising a substrate layer and superabsorbent particles, said apparatus comprising:
- substrate layer supplying means configured to provide an essentially endless substrate layer of fibrous non-woven material which is heat sensitive;
- a first heat application unit configured to apply heat to said substrate layer; and
- a particle depositing unit configured to deposit superabsorbent particles on said substrate layer.

Preferably, the heat sensitive non-woven material of the substrate layer is particularly suited to have its specific volume increased by application of heat. In other words, preferably the fibrous non-woven material is selected in such a way that it has a high relofting capability such that upon being heated, fibres of the material can easily open up and create additional volume, space and/or cavities in which superabsorbent particles can be deposited. When the material then cools down, the fibres and/or cavities created therein will close at least partially such that superabsorbent particles are retained within the cavities in between the fibres of the substrate layer. The relofting capability of a material is dependent on at least one of a chemical composition of the material, a spatial shape (of the fibres) of the material, a curvature (of the fibres) of the material, type of fibres, composition of fibres, etc.

According to an embodiment the first heat application unit comprises a first hot air device which is positioned upstream of the particle depositing unit and is configured to direct hot air towards the substrate layer.

According to an embodiment the hot air device is configured to provide hot air with a temperature between 60°C and 200°C, preferably between 80°C and 180°C, more preferably between 100°C and 160°C and most preferably between 120°C and 150°C.

According to an embodiment the hot air device is configured to direct hot air towards the substrate layer from a distance of 1 to 20 cm, preferably 2 to 15cm, more preferably 3 to 12 cm, and most preferably 5 to 10cm from the substrate layer.

According to an embodiment the apparatus further comprises a rotating member configured to guide the substrate layer along the rotating member at a speed of at least 1 m/s, preferably at least 2 m/s, more preferably at least 3 m/s, and most preferably at least 3,5 m/s.

According to an embodiment the hot air device is configured to direct hot air towards a surface of the substrate layer having a width of at least 50%, preferably at least 60%, more preferably at least 70%, even more preferably 80% and most preferably at least 90% of the width of the substrate layer. In this manner, it can be assured that a sufficiently large surface on which superabsorbent particles will be deposited is properly heated. The width of the substrate layer depends on the intended use of the substrate layer. In other words, substrate layers to be used in baby diapers may have a smaller width as compared to substrate layers to be used in adult incontinence article. Typically a width of a substrate layer for a baby diaper may be between 5 and 15 cm, depending on the size of the baby diaper, whereas a width of a substrate layer for an adult incontinence article may be comprised between 8 and 22cm, approximately. According to an embodiment the hot air device comprises a nozzle configured to blow the hot air towards the surface of the substrate layer, wherein the nozzle has a width corresponding to at least 50%, preferably at least 60%, more preferably at least 70%, even more preferably 80% and most preferably at least 90% of the width of the substrate layer. A preferred length of the nozzle depends on the speed of the substrate layer, thickness of the substrate layer, material of the substrate layer and distance between a nozzle end of the hot air device and the substrate layer. Exemplary lengths may be between 1cm and 50cm, preferably between 5cm and 40 cm, and more preferably between 10cm and 30cm.

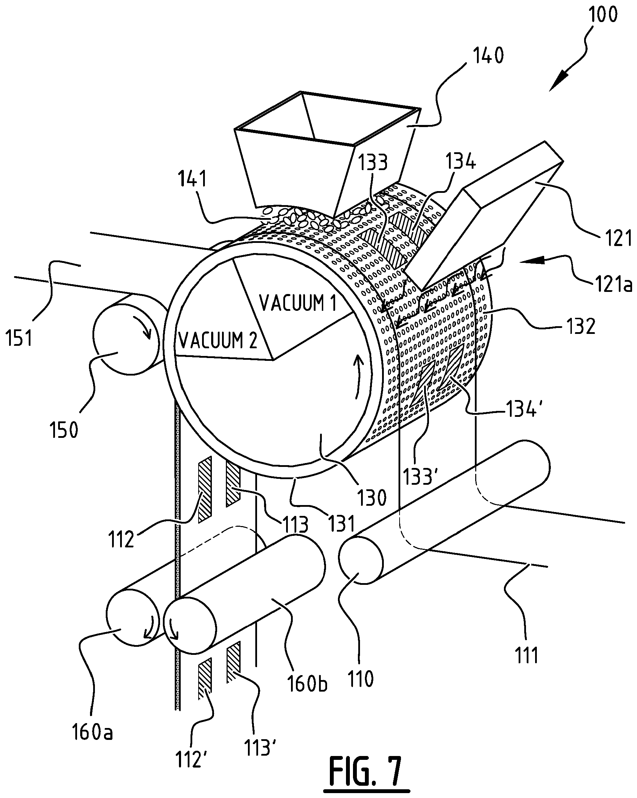

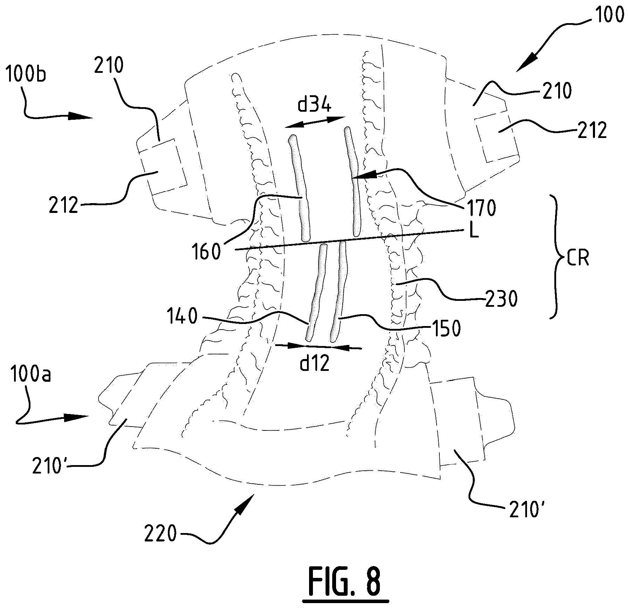

In an embodiment, the hot air device is configured to direct hot air towards multiple surfaces of the substrate layer, wherein the multiple surface are separated by a surface on which substantially no superabsorbent particles are to be deposited. To this end, the hot air device may be equipped with a plurality of precision nozzles wherein each precision nozzle is configured to direct hot air to a surface of the multiple surfaces. In this manner, areas where substantially no superabsorbent material is present can be created in the substrate layer and in the eventual absorbent structure. Alternatively or in addition, areas where substantially no superabsorbent material is present can be created in the substrate layer and in the eventual absorbent structure by depositing the superabsorbent particles onto and into the substrate layer in a controlled manner. A preferred embodiment of manufacturing an absorbent structure comprising areas where substantially no superabsorbent material is present will be elaborated in more detail in view of figures 7 and 8.

According to an embodiment the first heat application unit comprises a heating chamber which is positioned upstream of the particle depositing unit and comprises guiding means configured to guide the substrate layer through the heating chamber.

According to an embodiment the temperature in the heating chamber is between 60°C and 160°C, preferably between 70°C and 140°C, more preferably between 80°C and 120°C, and most preferably between 90°C and 110°C.

According to an embodiment the guiding means are configured to guide the substrate layer through the heating chamber at a speed of at least 1 m/s, preferably at least 2 m/s, more preferably at least 3 m/s, and most preferably at least 3,5 m/s.

According to an embodiment the guiding means are configured to guide the substrate layer through the heating chamber for a distance of 1 to 30m, preferably 2 to 25m, more preferably 3 to 20m, and more preferably 5 to 15m.

According to an embodiment the apparatus further comprises a second heat application unit positioned downstream of the particle depositing unit and configured to apply heat to said substrate layer after superabsorbent particles have been deposited thereon.

According to an embodiment the apparatus further comprises a first combing unit which is positioned upstream of the particle depositing unit and is configured to comb through the substrate layer.

According to an embodiment the apparatus further comprises a second combing unit which is positioned downstream of the particle depositing unit and is configured to comb through the substrate layer.

According to a preferred embodiment the apparatus further comprises:
- top layer supplying means configured to provide an essentially endless top layer such that the top layer is applied on top of the substrate layer;
- an attachment unit configured to attach said substrate layer to said top layer, enclosing the superabsorbent particles therebetween.

According to an embodiment the apparatus further comprises a sucking unit configured to suck the superabsorbent particles into the substrate layer after the superabsorbent particles are deposited on the substrate layer.

According to an embodiment the apparatus further comprises and adhesive providing unit configured to provide an adhesive to the substrate layer.

According to an embodiment the adhesive providing unit is configured to spray glue onto the substrate layer before and/or after superabsorbent particles are deposited on said substrate layer.

### BRIEF DESCRIPTION OF FIGURES

The accompanying drawings are used to illustrate presently preferred non-limiting exemplary embodiments of devices of the present invention. The above and other advantages of the features and objects of the invention will become more apparent and the invention will be better understood from the following detailed description when read in conjunction with the accompanying drawings, in which:
Figure 1 is a top plan view of an absorbent article, more in particular a diaper comprising an absorbent structure which is manufactured according to an embodiment of a method according to the present invention;
Figure 2 provides a cross-sectional schematic illustration of an absorbent structure which is manufactured according to an embodiment of the method according to the current invention;
Figure 3 provides a cross-sectional schematic illustration of the absorbent structure as illustrated in figure 2, further comprising a bottom layer beneath the substrate layer;
Figure 4 provides a cross-sectional schematic illustration of an absorbent structure which is manufactured according to an embodiment of the method according to the current invention, wherein the absorbent structure comprises comprising a substrate layer with superabsorbent particles, a release layer and an acquisition layer above the substrate layer;
Figure 5 illustrates an embodiment of the apparatus for manufacturing an absorbent structure comprising a substrate layer and superabsorbent particles according to the invention;
Figure 6 illustrates another embodiment of the apparatus for manufacturing an absorbent structure comprising a substrate layer and superabsorbent particles according to the invention;
Figure 7 illustrates another embodiment of the apparatus for manufacturing an absorbent structure comprising a substrate layer and superabsorbent particles according to the invention;
Figure 8 illustrates another embodiment of an exemplary embodiment of an absorbent article comprising an absorbent structure comprising a substrate layer and superabsorbent particles according to the invention, wherein the absorbent core comprises longitudinal channels which are substantially free of superabsorbent particles;
Figure 9 illustrates yet another embodiment of the apparatus for manufacturing an absorbent structure comprising a substrate layer and superabsorbent particles according to the invention;
Figure 10 illustrates an alternative embodiment of the apparatus for manufacturing an absorbent structure comprising a substrate layer and superabsorbent particles according to the invention; and
Figure 11 illustrates an exemplary embodiment of the apparatus for manufacturing an absorbent structure comprising a substrate layer and superabsorbent particles according to the invention.

### DESCRIPTION OF EMBODIMENTS

The present invention concerns a method and apparatus for manufacturing an absorbent structure which comprises an substantially cellulose free and/or essentially fluffless substrate layer and absorbent particles. Preferably, an underlying release structure is provided, thereby reducing eventual functional and structural problems associated with the absorbent articles and allowing the absorption, distribution and/or retention capacity of the absorbent structure to be more optimally used. The absorbent structure may comprise one or more absorbent layers, components, elements and/or inserts, wherein the absorbent core and/or release structure may comprise one or more absorbent polymer materials and/or areas, such as for instance absorbent particulate and/or fibrous polymer materials, displaying relevant absorbency, absorption (rates) and/or adsorption (rates), capillary action, mass flow and so. Next to the substrate layer and the optional release structure the absorbent structure may comprise additional coverstock, acquisition layers and/or dispersion layers, which layer(s) may or may not be attached to one another, the chassis and/or the absorbent article by mechanical, thermal, physical, chemical, thermo-mechanical and/or ultrasonic bond strength and cohesion. The absorbent structure comprises an absorbent core fibrous substrate layer which is essentially cellulose free and/or essentially fluffless, is sensitive to heat and contains absorbent polymer material, the release structure may comprise a web, matt, bat, nonwoven, woven, paper, tissue, knitted, tufted, stich-bonded, felted, films, airlaid, drylaid, wetlaid, spunlaid, spunlaced, meltblown, carded, staple, cellulose, wood pulp, fluff, curly fibers, fabrics, fibers, fabrics suitable to obtain desired structural and functional properties in accordance with the invention. Examples of suitable absorbent materials include creped cellulose wadding; melt blown polymers; chemically stiffened, modified or cross-linked cellulosic fibers; tissue, including tissue wraps and tissue laminates; absorbent foams; absorbent sponges; absorbent polymer materials; absorbent gelling materials; or any other known absorbent materials or combinations of materials. The use of cellulose fibers and/or fluff pulp is encompassed. The absorbent structure may further comprise minor amounts of non-liquid absorbent materials, such as adhesives, binders, plastics, waxes, oils and the like.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "an edge barrier" refers to one or more than one edge barrier.
"About" as used herein referring to a measurable value such as a parameter, an amount, a temporal duration, and the like, is meant to encompass variations of +/-20% or less, preferably +/-10% or less, more preferably +/-5% or less, even more preferably +/-1% or variations are appropriate to perform in the disclosed invention. However, it is to be understood that the value to which the modifier "about" refers is itself also specifically disclosed.
"Absorbent article", "absorbent garment", "absorbent product", "absorbing article", "absorbing garment", "absorbing product" and the like as used herein are used interchangeably and refer to devices that absorb and contain bodily exudates, and more specifically, refers to devices that are placed against or in proximity to the body of the wearer to absorb and contain the various liquids discharged from the body. Absorbent articles include but are not limited to feminine hygiene garments, baby diapers and pants, adult incontinence garments, various diaper and pants holders, liners, towels, absorbent inserts and the like.
"Absorbent core" as used herein refers to a three-dimensional part of the absorbent structure, comprising liquid-absorbing material, useful to permanently absorb and/or retain bodily exudates.
"Absorbent component" as used herein refers to a structural constituent of an absorbent article, e.g., a piece of an absorbent core, such as one of multiple pieces in a multi-piece absorbent core.
"Absorbent element" as used herein refers to a part of a functional constituent of an absorbent structure, e.g., a acquisition layer, a dispersion layer, core layer or a release structure formed of a material or materials having particular liquid handling characteristics suitable for the specific function.
"Absorbent fibrous polymer material" as used herein refers to an absorbent polymer material which is in threadlike from such as fibers, filaments, and the like so as to be less flowable in the dry state than particulates.
"Absorbent insert" as used herein refers to a device adapted for insertion into an "Absorbent layer" as used herein refers to a term referring to a discrete, identifiable sheet-like or web-like element of an absorbent article which may remain detached and relatively movable with respect to another such element or may be attached or joined so as to remain permanently associated with another such element. Each absorbent layer may itself include a laminate or combination of several layers, sheets and/or webs of similar or diverse compositions.
"Absorbent polymer material", "absorbent gelling material", "AGM", "superabsorbent", "superabsorbent material", "super absorbent polymer", "SAP" and the like as used herein are used interchangeably and refer to any suitable particulate (e.g., flaked, particulate, granular, or powdered) or fibrous cross linked polymeric materials that can absorb at least 5 times and preferably at least about 10 times or more its weight of an aqueous 0.9% saline solution as measured using the Centrifuge Retention Capacity test (EDANA 441.2-01).
"Absorbent polymer material area" as used herein refers to the area of the absorbent structure wherein adjacent layers are separated by a multiplicity of absorbent polymer material. Incidental contact areas between these adjacent layers within the absorbent particulate polymer material area may be intentional (e.g bond area's) or unintentional (e.g. manufacturing artifacts).
"Absorbent particulate polymer material" as used herein refers to an absorbent polymer material which is in particulate form such as powders, granules, flakes and the like so as to be flowable in the dry state.
"Absorption" as used herein refers to the process by which a liquid is taken up within a material.
"Absorption rate" as used herein refers to the rate of absorption of liquid, i.e. the amount of liquid which is absorbed per unit of time, typically by an absorbent component, element and/or absorbent layer of the absorbent article, structure and/or core.
"Acquisition layer", "acquisition region", "acquisition surface" or "acquisition material" and the like as used herein refer to the layer overlying the absorbent core having a faster liquid uptake and/or distribution capability.
"Absorbency" is the ability of a material to take up fluids by various means including capillary, osmotic, solvent, chemical and/or other action.
"Adult incontinence garment" as used herein refers to absorbent articles intended to be worn by incontinent adults, for absorbing and containing bodily exudates.
"Adhesion" as used herein refers to the force that holds different materials together at their interface.
"Adhesive" as used herein refers to a material, which may or may not be flowable in solution or when heated, that is used to bond materials together.
"Adsorption" as used herein refers to the process by which a liquid is taken up by the surface of a material.
"Airlaying" as used herein refers to forming a web by dispersing fibers or particles in an air stream and condensing them from the air stream onto a moving screen by means of a pressure and/or vacuum; a web of fibers produced by airlaying is herein referred to an "airlaid"; an airlaid web bonded by one or more techniques to provide fabric integrity is herein referred to an "airlaid nonwoven".
"Apparent density", "density" as used herein refers to the basis weight of the sample divided by the caliper with appropriate unit conversions incorporated therein. Apparent density used herein has the unit g/cm³.
"Attach", "attached" and "attachment" as used herein are synonymous with their counterparts of the terms "fasten", "affix", "secure", "bind", "join" and "link".
"Baby diaper" as used herein refers to absorbent articles intended to be worn by children, for absorbing and containing bodily exudates which the user draws up between the legs and fastens about the waist of the wearer.
"Baby pants" as used herein refers to absorbent articles marketed for use in transitioning children from diapers to underwear intended to cover the lower torso of children, so as to absorb and contain body exudates which article is generally configured like a panty garment and manufactured with a completed waist encircling portion, thereby eliminating the need for the user to fasten the article about the waist of the wearer.
"Back region" as used herein refers to the portion of an absorbent article or part thereof that is intended to be positioned proximate the back of a wearer.
"Backing" as used herein refers to a web or other material that supports and reinforces the back of a product.
"Basis weight" is the weight per unit area of a sample reported in grams per square meter, g/m² or gsm.
"Bodily exudates", "body exudates", "bodily fluids", "body fluids", "bodily discharges", "body discharges", "fluid(s)", " liquid(s)", "fluid(s) and liquid(s) and the like as used herein are used interchangeably and refer to, but are not limited to urine, blood, vaginal discharges, breast milk, sweats and fecal matter.
"Binder", "adhesive", "glue", "resins", "plastics" and the like as used herein are used interchangeably and refer to substances, generally in a solid form (e.g. powder, film, fiber) or as a foam, or in a liquid form (e .g. emulsion, dispersion, solution) used for example by way of impregnation, spraying, printing, foam application and the like used for attaching or bonding functional and/or structural components, elements and materials, for example including heat and/or pressure sensitive adhesives, hot-melts, heat activated adhesives, thermoplastic materials, chemical activated adhesives/solvents, curable materials and the like.
"Bond strength" as used herein refers to the amount of adhesion between bonded surfaces. It is a measure of the stress required to separate a layer of material from the base to which it is bonded.
"Capillary action", "capillarity", or "capillary motion" and the like as used herein are used to refer to the phenomena of the flow of liquid through porous media.
"Chassis" as used herein refers to a foundational constituent of an absorbent article upon which the remainder of the structure of the article is built up or overlaid, e.g., in a diaper, the structural elements that give the diaper the form of briefs or pants when configured for wearing, such as a backsheet, a topsheet, or a combination of a topsheet and a backsheet.
"Cellulose fibers" as used herein refers to naturally occurring fibers based on cellulose, such as, for example cotton, linen, etc; wood pulp fibers are one example of cellulose fibers; man-made fibers derived from cellulose, such as regenerated cellulose (rayon), or partially or fully acetylated cellulose derivatives (e.g. cellulose acetate or triacetate) are also considered as cellulose fibers.
"Cluster" or the like as used herein refers to an agglomeration of particles and/or fibers.
"Chemically stiffened fibers", chemically modified fibers", "chemically cross-linked fibers", "curly fibers" and the like as used herein are used interchangeably and refer to any fibers which have been stiffened by chemical means to increase stiffness of the fibers under both dry and aqueous conditions, for example by way of addition of chemical stiffening agents (e.g. by coating, impregnating, etc), altering the chemical structure of the fibers themselves (e.g. by cross-linking polymer chains, etc) and the like.
"Cohesion" as used herein refers to the resistance of similar materials to be separated from each other.
"Compartment" as used herein refers to chambers, cavities, pockets and the like.
"Comprise," "comprising," and "comprises" and "comprised of" as used herein are synonymous with "include", "including", "includes" or "contain", "containing", "contains" and are inclusive or open-ended terms that specify the presence of what follows e.g. a component and do not exclude or preclude the presence of additional, non-recited components, features, elements, members, steps, known in the art or disclosed therein.
"Coverstock" as used herein refers to a lightweight non-woven material used to contain and conceal an underlying absorbent core material; examples are the facing layer or materials that cover the absorbent cores of feminine hygiene garment s, baby diapers and pants and adult incontinence garments.
"Crotch region" of an absorbent article as used herein refers to about 50% of the absorbent article's total length (i.e., in the y-dimension), where the crotch point is located in the longitudinal center of the crotch region. That is, the crotch region is determined by first locating the crotch point of the absorbent article, and then measuring forward and backward a distance of 25% of the absorbent article's total length.
"Cross direction (CD)", "lateral" or "transverse" and the like as used herein are used interchangeably and refer to a direction which is orthogonal to the longitudinal direction and includes directions within ±45° of the transversal direction.
"Curing" as used herein refers to a process by which resins, binders or plastics are set into or onto fabrics, usually by heating, to cause them to stay in place; the setting may occur by removing solvent or by cross-linking so as to make them in soluble.
"Diaper", "conventional diaper", "diaper-like", "diaper-like garment" and the like as used herein are used interchangeably and refer to disposable absorbent articles, which typically include a front waist portion and a back waist portion which may be releasable connected about the hips of the wearer during use by conventional fasteners such as adhesive tape fasteners or hook and loop type fasteners. In use, the article is positioned between the legs of the wearer and the fasteners are releasable attached to secure the back waist portion to the front waist portion of the diaper, thereby securing the diaper about the waist of the wearer. The front waist portion and a back waist portion are connected by relatively non-stretchable or stretchable members (the term "stretchable" as used herein refers to materials that are extensible when forces are applied to the material, and offer some resistance to extension). Hence, such articles are generally not configured to be pulled up or down over the hips of the wearer when the fasteners are attached.
"Dispersion layer", "dispersion region", "dispersion surface" or "dispersion material" and the like as used herein refer to the layer overlying the absorbent core having a faster liquid uptake and dispersion capability.
"Disposable" is used herein to describe articles that are generally not intended to be laundered or otherwise restored or reused (i.e., they are intended to be discarded after a single use and, preferably, to be recycled, composted or otherwise disposed of in an environmentally compatible manner).
"Drylaying" as used herein refers to a process for making a nonwoven web from dry fiber; these terms apply to the formation of carded webs, as well as to the air laying formation of random webs; a web of fibers produced by drylaying is herein referred to as a "drylaid"; a drylaid web bonded by one or more techniques to provide fabric integrity is herein referred to a "drylaid nonwoven".
"Dry strength" as used herein refers to the strength of ajoint determined in dry state conditions, immediately after drying under specified conditions or after a period of conditioning in the standard laboratory atmosphere.
"Essentially cellulose free" or "little to no cellulose fibers" as used herein refers to an absorbent article, structure, core component and/or element containing less than 20% by weight cellulosic fibers, less than 10% cellulosic fibers, less than 5% cellulosic fibers, no cellulosic fibers, or no more than an immaterial amount of cellulosic fibers which do not materially affect the thinness, flexibility or absorbency thereof.
"Essentially fluffless" or "little to no fluff pulp" as used herein refers to an absorbent article, structure, core, component and/or element containing less than 20% by weight fluff pulp, less than 10% fluff pulp, less than 5% fluff pulp, no fluff pulp, or no more than an immaterial amount of fluff pulp which do not materially affect the thinness, flexibility or absorbency thereof.
"Fabric" as used herein refers to a sheet structure made from fibers, filaments and/or yarns.
"Feminine hygiene garments" as used herein refer to absorbent hygiene articles intended to be worn by woman, for absorbing and containing body exudates.
"Fiber" as used herein refers to the basic threadlike structure from which nonwovens, yarns and textiles are made. It differs from a particle by having a length at least 4 times its width; "Natural fibers" are either of animal (wool, silk), vegetable (cotton, flax, jute) or mineral (asbestos) origin, while "Man-made fibers" may be either polymers synthesized from chemical compounds (polyester, polypropylene, nylon, acrylic etc.) or modified natural polymers (rayon, acetate) or mineral (glass). "Fiber" and "filament" are used interchangeably.
"Fluff pulp" or "Pulp fluff" as used herein refers to wood pulp specially prepared to be drylaid. The fibers can be either natural or synthetic or a combination thereof.
"Front region" as used herein refers to the portion of an absorbent article or part thereof that is intended to be positioned proximate the front of a wearer.
"Garment facing layer" as used herein refers to elements of the chassis that form the outer surface of the absorbent article, such as the backsheet, the side panels, the waist fasteners, and the like, when such elements are present.
"Heat activated adhesive" as used herein refers to a dry adhesive that is rendered tacky or fluid by application of heat or heat and pressure to the assembly.
"Heat sealing adhesive" as used herein refers to a thermoplastic adhesive which is melted between the adherent surfaces by heat application to one or both of the adjacent adherent surfaces.
"High loft" as used herein refers to general term of low density, thick or bulky fabrics.
"Hot-melt adhesive" as used herein refers to a solid material that melts quickly upon heating, then sets to a firm bond upon cooling; used for almost instantaneous bonding.
"Hydrophilic" as used herein refers to having an affinity for being wetted by water or for absorbing water.
"Hydrophobic" as used herein refers to lacking the affinity for being wetted by water or for absorbing water.
"Immobilization layer" as used herein refers to a layer able to be applied to the absorbent polymer material or absorbent polymer material area with the intent to gather, bond and/or immobilize absorbent material and/or absorbent layer.
"Join", "joined" and "joining" as used herein refers to encompassing configurations wherein an element is directly secured to another element by affixing the element directly to the other element, as well as configurations wherein the element is indirectly secured to the other element by affixing the element to an intermediate member or members which in turn is or are affixed to the other element.
"Knitting" as used herein refers to the technique for interlocking loops of fibers with needles or similar devices.
"Layer" refers to identifiable components of the absorbent article, and any part referred to as a "layer" may actually comprise a laminate or combination of several sheets or webs of the requisite type of materials. As used herein, the term "layer" includes the terms "layers" and "layered." "Upper" refers to the layer of the absorbent article which is nearest to and/ or faces the wearer facing layer; conversely, the term "lower" refers to the layer of the absorbent article which is nearest to and/or faces the garment facing layer. "Layer" is three dimensional structure with a x dimension width, y dimension length, and z-dimensions thickness or caliper, said x-y dimensions being substantially in the plane of the article, however it should be noted that the various members, layers, and structures of absorbent articles according to the present invention may or may not be generally planar in nature, and may be shaped or profiled in any desired configuration .
"Machine direction (MD)", "longitudinal" and the like as used herein are used interchangeably and refer to a direction running parallel to the maximum linear dimension of the structure and includes directions within ±45° of the longitudinal direction.
"Major surface" as used herein refers to a term used to describe the surfaces of greatest extent of a generally planar or sheet-like structural element and to distinguish these surfaces from the minor surfaces of the end edges and the side edges, i.e., in an element having a length, a width, and a thickness, the thickness being the smallest of the three dimensions, the major surfaces are those defined by the length and the width and thus having the greatest extent.
"Mass flow" as used herein refers to the f low of a liquid f rom one absorbent element or component to another absorbent element or component by channel flow action.
"Mechanical bonding" as used herein refers to a method of bonding fibers by entangling them. This can be achieved by needling, stitching with fibers or by the use of high-pressure air or water jets and the like.
"Nonwoven" as used herein refers to manufactured sheet, web or batt of directionally or randomly orientated fibers, bonded by friction, and/or cohesion and/or adhesion, excluding paper and products which are woven, knitted, tufted, stitch-bonded incorporating binding yarns or filaments, or felted by wet-milling, whether or not additionally needled. The fibers may be of natural or man-made origin and may be staple or continuous filaments or be formed in situ. Commercially available fibers have diameters ranging from less than about 0.001 mm to more than about 0.2 mm and they come in several different forms: short fibers (known as staple, or chopped), continuous single fibers (filaments or monofilaments), untwisted bundles of continuous filaments (tow), and twisted bundles of continuous filaments (yarn). Nonwoven fabrics can be formed by many processes such as melt blowing, spun bonding, solvent spinning, electrospinning, and carding. The basis weight of nonwoven fabrics is usually expressed in grams per square meter (gsm).
"Pant", "training pant", "closed diapers", "prefastened diapers", "pull-on diapers" and "diaper-pants" and the like as used herein are used interchangeably and refer to absorbent articles which are typically applied to the wearer by first leading the feet into the respective leg openings and subsequently pulling the pants from the feet to waist area over the hips and buttocks of the wearer and which are capable of being pulled up or down over the hips of the wearer. Typically, such articles may include a front waist portion and a back waist portion which may be connected about the hips of the wearer by integral or releasable members. A pant may be preformed by any suitable technique including, but not limited to, joining together portions of the article using refastenable and/or nonrefastenable bonds (e.g., seam, weld, adhesive, cohesive bond, fastener, etc.). A pant may be preformed anywhere along the circumference of the article (e.g., side fastened, front waist fastened).
"Polymer" as used herein refers to but is not limited to, homopolymers, copolymers, such as for example, block, graft, random and alternating copolymers, terpolymers, etc. and blends and modifications thereof. Unless otherwise specifically limited, the term "polymer" includes all possible spatial configurations of the molecule and include, but are not limited to isotactic, syndiotactic and random symmetries.
"Rear" as used herein refers to the portion of an absorbent article or part thereof that is intended to be positioned proximate the back of the wearer.
"Release structure", "release region", "release surface" or "release material" and the like as used herein are used interchangeably and refer to a structure in fluid communication with the absorbent core having a larger relative liquid absorption capacity and/or rate allowing it to quickly take up, temporarily hold and releasing liquids.
"Resin" as used herein refers to a solid or semisolid polymeric material.
"Thermobonding" as used herein refers to a method of bonding fibers by the use of heat and/or high-pressure.
"Thermoplastic" as used herein refers to polymeric materials that have a melting temperature and can flow or be formed into desired shapes on the application of heat at or below the melting point.
"Ultrasonic" as used herein refers to the use of high frequency sound to generate localized heat through vibration thereby causing thermoplastic fibers to bond to one another.
"Water-absorbing", "liquid-absorbing", "absorbent", "absorbing" and the like as used herein are used interchangeably and refer to compounds, materials, products that absorb at least water, but typically also other aqueous fluids and typically other parts of bodily exudates such as at least urine or blood.
"Wearer facing layer" as used herein refers to elements of the chassis that form the inner surface of the absorbent article, such as the topsheet, the leg cuffs, and the side panels, etc., when such elements are present.
"Weaving" as used herein refers to the process of interlacing two or more sets of yarns at right angles to form a fabric; a web of fibers produced by weaving is herein referred to as a "woven".
"Web material" as used herein refers to an essentially endless material in one direction, i.e. the longitudinal extension or the length, or the x- direction in Cartesian coordinates relative to the web material. Included in this term is an essentially unlimited sequence of pieces cut or otherwise separated from an essentially endless material. Often, though not necessarily, the web materials will have a thickness dimension (i.e. the z-direction) which is significantly smaller than the longitudinal extension (i.e. in x-direction). Typically, the width of web materials (they-direction) will be significantly larger than the thickness, but less than the length. Often, though not necessarily, the thickness and the width of such materials is essentially constant along the length of the web. Without intending any limitation, such web materials may be cellulosic fiber materials, tissues, woven or nonwoven materials and the like. Typically, though not necessarily, web materials are supplied in roll form, or on spools, or in a folded state in boxes. The individual deliveries may then be spliced together to form the essentially endless structure. A web material may be composed of several web materials, such as multilayer non-woven, coated tissues, nonwoven/film laminates. Web materials may comprise other materials, such as added binding material, particles, hydrophilizing agents and the like.
"Wet burst strength" is a measure of a layer's ability to absorb energy, when wet and subjected to deformation normal to the plane of the web.
"Wet strength" as used herein refers to the strength of a joint determined immediately after removal from a liquid in which it has been immersed under specified conditions of time, temperature and pressure. The term is commonly used in the art to designate strength after immersion in water.
"Wetlaying" as used herein refers to the forming a web from an aqueous dispersion of fibers by applying modified paper making techniques; a web of fibers produced by wetlaying is herein referred to as a "wetlaid".
"Wood pulp" as used herein refers to cellulosic fibers used to make viscose rayon, paper and the absorbent cores of products such as feminine hygiene garments, baby diapers and pants and adult incontinence garments.
"X-y dimension" as used herein refers to the plane orthogonal to the thickness of the article, structure or element. The x- and y-dimensions correspond generally to the width and length, respectively, of the article, structure or element.
"Z-dimension" as used herein refers to the dimension orthogonal to the length and width of the article, structure or element. The z-dimension corresponds generally to the thickness of the article, structure or element.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

The same or similar features and components are indicated with the same reference numerals throughout the figures.

The recitation of numerical ranges by endpoints includes all numbers and fractions subsumed within that range, as well as the recited endpoints.

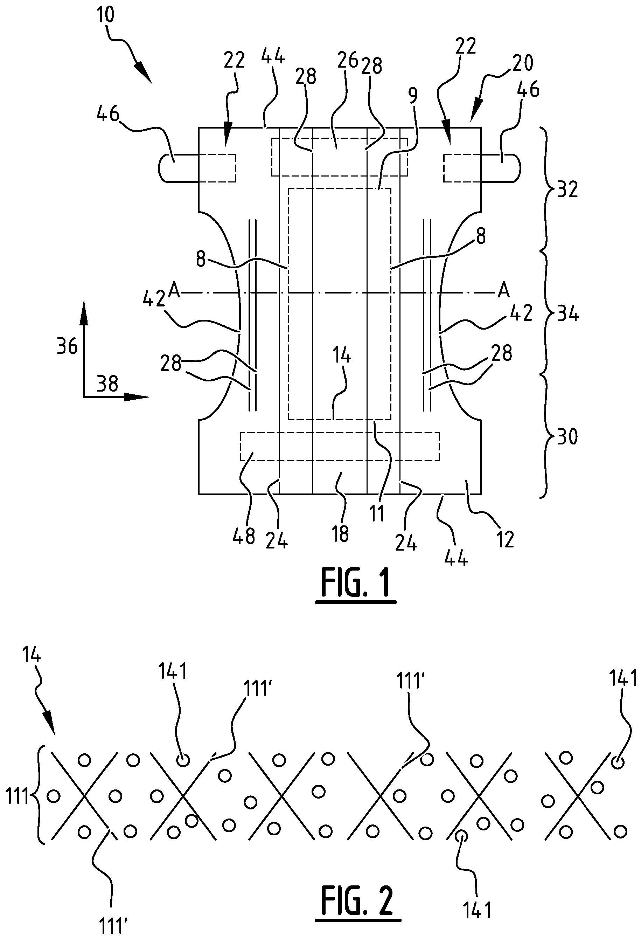

Figure 1 is a top plan view of a diaper 10 with an absorbent structure 14 as manufactured according to a preferred embodiment of the method according to the present invention. It should be understood, however, that the present invention is also applicable to other absorbent articles such as feminine hygiene garments, baby pants, adult incontinent garments and the like.

The absorbent article is shown in its flat out, un-contracted state with the wearer side facing the viewer. The chassis 12 of the diaper 10 in Figure 1 comprises the main body of the diaper 10. The chassis 12 comprises an outer covering including a liquid pervious topsheet 18 and/or a liquid impervious backsheet 20. The chassis 12 may include a portion of an essentially fluffless absorbent structure 14 encased between the topsheet 18 and the backsheet 20. The chassis 12 may also include most or all of the absorbent structure 14 encased between the topsheet 18 and the backsheet 20. The chassis 12 preferably further includes side panels or ears 22, elasticized leg cuffs 28 and elastic waist features 26. One end portion of the diaper 10 is configured as a front waist region 30 of the diaper 10. The opposite end portion is configured as a back waist region 32 of the diaper 10. An intermediate portion of the diaper 10 is configured as a crotch region 34, which extends longitudinally between the first and second waist regions 30 and 32. The waist regions 30 and 32 may include elastic elements such that they gather about the waist of the wearer to provide improved fit and containment (e.g. elastic waist feature 26). The crotch region 34 is that portion of the diaper 10 which, when the diaper 10 is worn, is generally positioned between the wearer's legs. The diaper 10 is depicted with its longitudinal axis 36 and its transverse axis 38. The periphery of the diaper 10 is defined by the outer edges of the diaper 10 in which the longitudinal edges 42 run generally parallel to the longitudinal axis 36 of the diaper 10 and the end edges 44 run between the longitudinal edges 42 generally parallel to the transverse axis 38 of the diaper. The chassis 12 also comprises a fastening system, which may include at least one fastening or securing member 46 and at least one landing zone 48. The various components within the diaper 10 may be bound, joined or secured by any method known in the art, for example by adhesives in uniform continuous layers, patterned layers or arrays of separate lines, spirals or spots. The topsheet 18, the backsheet 20, the absorbent structure 14 and other components may be assembled in a variety of well-known configurations and are well known in the art.

The backsheet 20 covers the absorbent structure 14 and preferably extends beyond the absorbent structure 14 toward the longitudinal edges 42 and end edges 44 of the diaper 10 and may be joined with the topsheet 18. The backsheet 20 prevents the bodily exudates absorbed by the absorbent structure 14 and contained within the diaper 10 from soiling other external articles that may contact the wearer, such as bed sheets and undergarments. In preferred embodiments, the backsheet 20 is substantially impervious to bodily exudates and comprises a laminate of a nonwoven and a thin plastic film such as a thermoplastic film. The backsheet 20 may comprise breathable materials that permit vapor to escape from the diaper 10 while still preventing bodily exudates from passing through the backsheet 20. It may be semi-rigid, non-elastic and can be made fully or partially elasticized and include backing. The backsheet 20 may be assembled in a variety of well-known configurations and are well known in the art.

The diaper 10 comprises a topsheet 18 that is preferably soft, compliant, exhibits good strikethroughs and has a reduced tendency to rewet from the liquid absorbent material. The topsheet 18 is placed in close proximity to the skin of the wearer when the diaper 10 is worn. In this way, such topsheet 18 permits bodily exudates to rapidly penetrate it so as to flow toward the absorbent structure 14 more quickly, but preferably not allowing such bodily exudates to flow back through the topsheet 18. The topsheet 18 may be constructed from any one of a wide range of liquid and vapor permeable, preferably hydrophilic, materials. The upper and lower surface of the topsheet 18 may be treated differently and may for instance include a surfactant on the upper surface so as to facilitate liquid transfer there through, especially at a central zone or area of the topsheet 18 located over the absorbent structure 14, and for instance include a hydrophobic agent on the lower surface to minimize the liquid contained within the absorbent core from contact wetting the topsheet 18 thereby reducing rewet values. The topsheet 18 may also be coated with a substance having rash preventing or rash reducing properties (e.g. aloe vera). The topsheet 18 covers substantially the entire wearer facing area of the diaper 10, including substantially all of the front waist region 30, back waist region 32, and crotch region 34. Further, the side panels 22 and/or waist feature layers of the inner region may be formed from the same single topsheet material and, thus, may be referred to as being unitary with the topsheet 18 in forming longitudinal and lateral extensions of the top sheet 18 material. Alternatively, the topsheet 18 may be formed from multiple different materials which vary across the width of the topsheet 18. Such a multiple piece design allows for creation of preferred properties and different zones of the topsheet 18. The topsheet 18 may be semi-rigid, non-elastic and can be made fully or partially elasticized. The topsheet 18 may be assembled in a variety of well-known configurations and are well known in the art.

The absorbent structure 14 in Figure 1 generally is disposed between the topsheet 18 and the backsheet 20. The absorbent structure 14 may comprise any absorbent material that is generally compressible, conformable, non-irritating to the wearer's skin, and capable of absorbing and retaining bodily exudates. The absorbent structure 14 may comprise a wide variety of liquid absorbent materials commonly used in absorbent articles. The absorbent structure 14 according to various embodiments of the invention may be configured to extend substantially the full length and/or width of the diaper 10. However, alternatively the absorbent structure 14 according to the invention is not coextensive with the entire diaper 10 and is limited to certain regions of the diaper 10 such as for instance the crotch region 34. In various embodiments, the absorbent structure 14 extends to the edges of the diaper 10 and the absorbent material is concentrated in the crotch region 34 or another target zone of the diaper 10. In still another embodiment, the particles can be a combination of absorbent material, preferably comprising absorbent polymer material, and skin care particles such as ion exchange resins, deodorant, anti-microbial agents, binder particles, or other beneficial particles. The absorbent structure 14 is displayed as having a substantially rectangular configuration, however, the absorbent structure can also comprise other shapes known in the art such as, elliptical shaped, dogbane shaped, T-shaped or I-shaped configurations.

The diaper 10 may also utilize a pair of containment walls or cuffs 24. Each cuff 24 is a longitudinally extending wall structure preferably positioned on each side of the absorbent structure 14 and spaced laterally from the longitudinal axis 36. The longitudinal ends of the cuffs 24 may be attached or joined, for example, to the topsheet 18 in the front and rear waist regions 30 and 32. Preferably, the ends of the cuffs 24 are tacked down inwardly and attached, for example, by adhesive or sonic bonding to the lower structure. Such a construction effectively biases the cuffs 24 inwardly and is generally considered to cause the cuffs 24 to exhibit improved leakage prevention properties. Preferably, the cuffs 24 are equipped with elastic members 28, which extend along a placed within the cuffs 24, preferably at the top of the cuff 24 while in a stretched condition and then glued or sonic bonded to the cuff 24 at least at their ends. When released or otherwise allowed relaxing, the elastic members 28 retract inwardly. When the diaper 10 is worn, the elastic members 28 function to contract the cuffs 24 about the buttocks and the thighs of the wearer in a manner, which forms a seals between the diaper 10, the buttocks and the thighs. The cuffs 24 may be assembled in a variety of well-known configurations and are well known in the art.

The diaper 10 may also employ additional layers including an acquisition layer and/or dispersion layer situated between the topsheet and the absorbent core and/or coverstock layers. This serves to slow down the flow so that the liquid has adequate time to be absorbed by the absorbent core.

In order to keep the diaper 10 in place about the wearer, preferably at least a portion of the back waist region 32 is attached by fastening or securing members 46 to at least a portion of the front waist region 30, preferably to form leg openings and an absorbent article waist. Fastening or securing members 46 carry the tensile load around the absorbent article waist and compliment the elastic members 28 by providing a quasi-seal between the wearer, the elastic waist feature 26 and cuffs 24, so that bodily exudates are contained within the diaper 10 which are then absorbed. In other words, so that it does not leak through gaps between the wearer and the edge of the diaper 10. The fastening or securing members 46 may for instance be adhesive, mechanical fasteners, hook and loop features, conceivable strings and/or combinations thereof, i.e., anything that will secure one end of the diaper 10 to the longitudinally opposite end of the diaper 10. The fastening or securing members 46 may also be co-adhesive such that they adhere to each other but not other materials. The fastening or securing members 46 and any component thereof may include any material suitable for such a use, including but not limited to plastics, films, foams, non-woven webs, woven webs, paper, laminates, fiber reinforced plastics and the like, or combinations thereof. It may be preferable that the materials making up the fastening or securing members 46 are flexible, extensible and/or elastic, allowing them to better conform to the shape and movements of the body and thus, reduces the likelihood that the fastening system will irritate or injure the wearer's skin. Preferably, the diaper 10 is affixed to the wearer by tape fasteners which are permanently affixed to the backsheet 20. Tape fasteners are contacted with the transversely opposite side panel or ears 22 attached or joined and extending from the backsheet 20, where they remain affixed due to the binding compound applied to the fasteners. Alternatively, the absorbent article may be pants and the like. In this configuration, the absorbent article may or may not have tape fasteners. Specific disposability tapes may however also be provided on such absorbent articles. All fastening and securing elements 46 may be assembled in a variety of well-known configurations and are well known in the art.

The waist regions 30 and 32 each comprise a central region and a pair of side panels or ears 22 which typically comprise the outer lateral portions of the waist regions. These side panels 22 may be unitary with the chassis 12 and/or backsheet 20 or may be attached or joined thereto by any means know in the art. In a preferred embodiment of the present invention, the side panels 22 positioned in the back waist region 32 are flexible, extensible and/or elastic in at least the lateral direction (i.e., elasticized side panels), in another embodiment the side panels 22 are non-elastic, semi-rigid, rigid and/or stiff. These varieties of side panels 22 are well known in the art.

Furthermore waistbands 26 employing elastic members can be positioned along the transverse portion of the diaper 10 so that when worn, the waistbands 26 are positioned along the waist of the wearer. Generally, the waistband 26 preferably creates a seal against the waist so that bodily exudates do not leak from the regions between the elastic waistband 26 and the waist of the wearer. Although the bodily exudates are primarily absorbed by the absorbent materials within the diaper 10, the seal is important considering the assault of liquid by the wearer may overwhelm the absorption rate capacity of the absorbent structure 14. Hence, the waistbands 26 contain the liquid while it is being absorbed, they are well known in the art.

The absorbent article such as a diaper 10 may also include such other features, components and elements as are known in the art including front and rear ear panels, waist cap features, elastics and the like to provide better fit, containment and aesthetic characteristics. These features may be assembled in a variety of well-known configurations and are well known in the art.

Figure 2 provides a cross-sectional schematic illustration of an absorbent structure 14 which is manufactured according to an embodiment of the method according to the current invention. The absorbent structure comprises a substrate layer 111 and superabsorbent particles 141 distributed within the substrate layer 111. For the sake of simplicity the absorbent structure 14 of figure 2 is shown as merely a substrate layer 111 wherein superabsorbent particles 141 are present within the fibres 111' of the substrate layer. But according to alternative embodiments of the present invention, a method and apparatus is provided to manufacture absorbent structures 14 which may further comprise a top layer positioned on top of the substrate layer 111, a bottom layer positioned underneath the substrate layer 111, a release structure, a coverstock, an edge barrier, an acquisition layer and/or a dispersion layer, which layer(s) may or may not be attached to one another, the chassis and/or the absorbent article by mechanical, thermal, physical, chemical, thermo-mechanical and/or ultrasonic bond strength and cohesion. In every embodiment the absorbent structure 14 comprises an absorbent core fibrous substrate layer 111 which is essentially cellulose free and/or essentially fluffless, is heat-sensitive and contains absorbent polymer material 141. Additional structures and/or layers may comprise a web, matt, bat, nonwoven, woven, paper, tissue, knitted, tufted, stich-bonded, felted, films, airlaid, drylaid, wetlaid, spunlaid, spunlaced, meltblown, carded, staple, cellulose, wood pulp, fluff, curly fibers, fabrics, fibers, fabrics suitable to obtain desired structural and functional properties in accordance with the invention. Although, due to the simplicity of the schematic drawing it may seem that superabsorbent particles 141 may fall trough the fibers of the substrate layer 111, it is clear to the skilled person that, in practice, the non-woven fibrous substrate layer 111 will be able to accommodate at least the majority of superabsorbent particles 141 deposited thereon and that the fibrous structure will keep the superabsorbent particles 141 from falling through the substrate layer 111. The heat sensitive non-woven material of the substrate layer 111 is particularly suited to have its specific volume increased by application of heat. In other words, the fibrous non-woven material is selected in such a way that it has a high relofting capability such that upon being heated, fibres 111' of the material can easily open up and create additional volume, space and/or cavities in which superabsorbent particles can be deposited. When the material then cools down, the fibres 111' and/or cavities created therein will close at least partially such that superabsorbent particles are retained within the cavities in between the fibres of the substrate layer. The relofting capability of a material is dependent on at least one of a chemical composition of the material, a spatial shape (of the fibres) of the material, a curvature (of the fibres) of the material, type of fibres, composition of fibres, etc. The substrate layer 111 material comprises at least two types (not shown) of fibres, binder fibres and carrier fibres. The binder fibres may consist of sheath-core bicomponent fibres and the carrier fibres consist of polyester fibres.

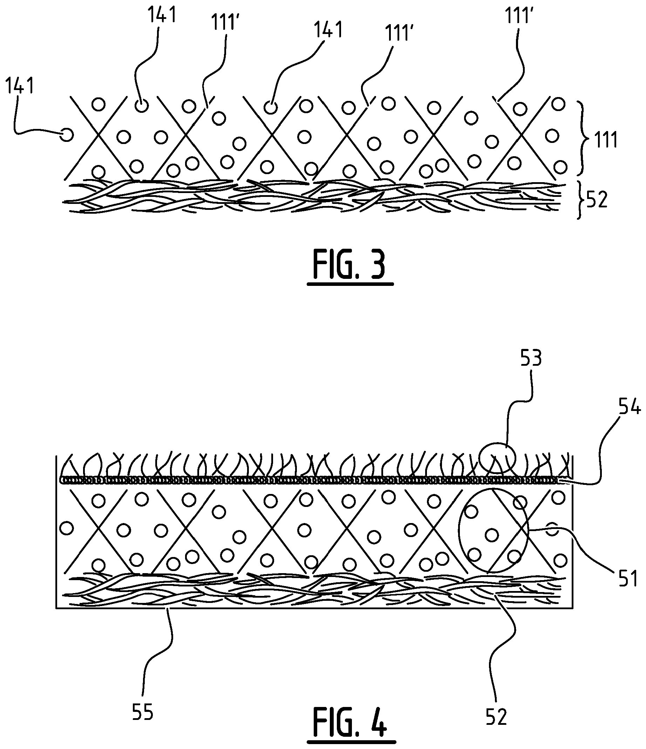

Figure 3 illustrates the absorbent structure 14 as shown in figure 2, with an additional bottom layer or carrier layer 52. This bottom layer 52 may be an integral part of the substrate layer 111 or may be a separate layer being attached to the substrate layer 111. Preferably the bottom layer 52 is provided to further avoid superabsorbent particles 141 to migrate or fall out of the substrate layer 111. According to an embodiment of the method according to the invention, the bottom layer 52 may be provided to the substrate layer 111 before superabsorbent particles 141 are deposited thereon. In alternative embodiments the bottom layer 52 may be provided to the substrate layer 111 after superabsorbent particles 141 are deposited thereon. It will be clear to the skilled person that the methods and apparatuses as disclosed below are not limited to the specific composition of the non-woven material. However, for purposes of illustration, the non-woven material may comprise a homogeneous blend of binder fibres and carrier fibres. In embodiments which are not within the scope of the claims, the binder fibres and/or the carrier fibres may be natural fibres. Polyester fibres, which are thermoplastic polymer fibres, are suitable synthetic carrier fibres. In exemplary embodiments which are not within the scope of the claims other thermoplastic polymer fibres such as polypropylene are suitable synthetic carrier fibres, whereas wool, cotton, and silk are examples of suitable natural carrier fibres. It is clear to the skilled person that other fibres can be used in the non-woven material depending upon the precise processing limitations imposed and the desired characteristics of the non-woven material and/or the resulting absorbent structure.

Figure 4 illustrates an absorbent structure according to an embodiment of the invention. From top to bottom, the absorbent structure comprises an acquisition layer 53, a dispersion layer 54, a substrate layer 51 with fibers and absorbent polymer materials, a bottom layer as release structure 52 and a wicking and edge barrier 55. The acquisition layer 53 and the dispersion layer 54 may be unitary or non-unitary with the absorbent core. Also the release structure 52 can be unitary or non-unitary with the absorbent core 51. Moreover, the release structure 52 may be unitary or non-unitary with the wicking and edge barrier 55. In other words, the substrate layer 51 may be complimented with an additional layer or layers at the top and/or bottom thereof to form an absorbent structure 14.

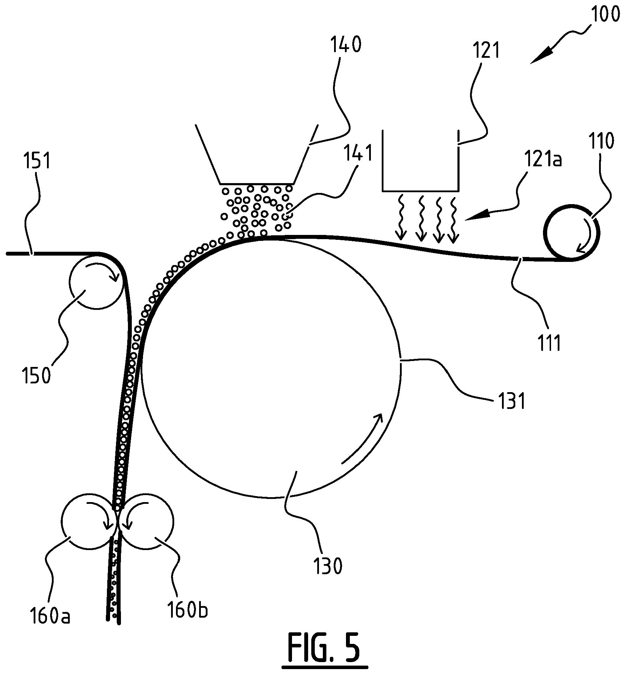

Figure 5 illustrates an embodiment of an apparatus 100 for manufacturing an absorbent structure comprising a substrate layer 111 and superabsorbent particles 141. The apparatus 100 comprises substrate layer supplying means 110 which are configured to provide an essentially endless substrate layer 111 of fibrous non-woven material which is heat sensitive. In the illustrated embodiment, the substrate layer supplying means is a reel 110 configured to feed a continuous tape or endless layer of substrate material towards an outer surface 131 of a transport device 130. It is clear to the skilled person that alternative substrate layer supplying means may be applied, for example the substrate layer may be formed in line. The transport device 130 is illustrated as a rotating member 130 and more in particular as a wheel rotating around its own central axis and having an outer surface 131. In alternative embodiments, alternative transport devices or rotating members may be used, such as a belt having a flat outer surface which is configured to carry the substrate layer 111. Further downstream, the apparatus 100 comprises a first heat application unit 121 configured to apply heat to said substrate layer 111. Heat may be applied in various manners. For example, the substrate layer 111 may be provided on a rotating member and guided through a heating chamber wherein a heating source such as an oven, burner, infrared lamp, hot air device produces heat which may be transferred to the substrate layer 111. Heat may be provided through hole of the rotating member, heat may be provided downward to the substrate layer, etc. When the temperature of the substrate layer 111 is increased the binder fibres will begin to soften and some of the bonder fibres and/or carrier fibres will position their selves approximately parallel with the softened binder fibres while detaching therefrom. This realigning and/or detaching action will cause the substrate layer 111 to increase in thickness as the specific volume thereof increases. In the illustrated embodiment the first heat application unit comprises a first hot air device 121, positioned upstream of a particle depositing unit 140. Preferably, the upstream first hot air device 121 is positioned such that it can apply hot air to the substrate layer 111 before the substrate layer 111 reaches the outer surface 131 of the transport device 130. In this manner, as shown in figure 5, superabsorbent particles 141 can be deposited on the substrate layer 111 directly after the substrate layer 111 has been heated by the first hot air device 121. Alternatively, the upstream first hot air device 121 is positioned above the outer surface 131 of the transport device 130 such that it can apply hot air to the substrate layer 111, while the substrate layer 111 is guided on the outer surface 131 of the transport device 130, such that superabsorbent particles 141 can be deposited on the substrate layer 111 directly after the substrate layer 111 has been heated by the first hot air device 121. The first hot air device is configured to generate and direct hot air 121a towards the substrate layer 111. By directing hot air 121a towards the heat sensitive substrate layer 111, fibres within the substrate layer 111 will curl under the influence of the hot air 121a heat which causes the substrate layer 111 to open up. This way, an available volume for accommodating superabsorbent particles 141 which are provided by the particle depositing unit 140 within the substrate layer is increased, without harming the fibers of the substrate layer 111 and without negatively affecting the wet and/or dry integrity of the absorbent structure. Furthermore, by opening up the fibrous substrate layer 111 by applying hot air 121a, a deep penetration of superabsorbent particles 141 within the substrate layer may be achieved, and an increased density of superabsorbent particles 141 being distributed within the substrate layer 111 is made possible. The extent to which the available volume within the substrate layer 111 increases will depend on the material of the fibrous substrate layer, the temperature to which the substrate layer 111 is exposed and the time during which the substrate layer 111 is exposed to the high temperature. Especially material characteristics such as a thickness of the substrate layer and/or a gsm of the substrate layer may influence the achieved effect of volume increase. Regarding temperature, it has been observed by the inventors that for most materials which are suitable to be used in the substrate layer 111 as described earlier, fibres will start to react and curl when heated to a temperature of approximately 60°C and that a maximal increase of specific volume is achieved when the fibres are heated to a temperature between 80°C and 120°C, preferably a temperature of approximately 100°C. For most of the suitable materials, fibres will become susceptible for melting and/or degradation when heated to temperatures which are higher than approximately 150°C. Taking into account a speed of the transport device 130, which is typically between 1m/s and 5m/s, which results in a relatively short exposure time of the substrate layer 111 to the hot air 121a from the hot air device 121, the hot air device is configured to generate hot air having a temperature which is preferably higher than the temperatures as mentioned above. Moreover, the generated hot air 121a which is directed towards the substrate layer 111 may have a temperature between 60°C and 200°C, preferably between 80°C and 180°C, more preferably between 100°C and 160°C and most preferably between 120°C and 150°C. The hot air device may be positioned such that the hot air 121a has to travel a distance of 1 to 20 cm, preferably 2 to 15cm, more preferably 3 to 12 cm, and most preferably 5 to 10cm from hot air device 121 to the substrate layer 111. The apparatus 100 further comprises the particle depositing unit 140 which is configured to deposit superabsorbent particles 141 onto said substrate layer 111. Because the substrate layer 111 is heated before superabsorbent particles 141 are provided, the fibers of the substrate layer 111 will curl, open up and increase an amount of available volume within the substrate layer 111 to accommodate the superabsorbent particles 141 which are to be provided by the particle depositing unit 140. In a preferred embodiment, the particle depositing unit 140 is configured to deposit measured quantities of superabsorbent particles 141 onto the substrate layer 111 in various manners. The superabsorbent granular material 141 may be deposited onto the substrate layer 111 in a uniform manner. However, in an alternative embodiment the particle depositing unit 140 is configured to deposit the superabsorbent particles 141 on the substrate layer 111 with non-uniform dosages in a longitudinal direction and/or in a transverse direction of the endless substrate layer 111, so that the superabsorbent particles 141 are distributed with different densities in different areas of the substrate layer in order to modulate and control the absorption characteristics of the various areas of the absorbent structure.

In an embodiment, the superabsorbent particles may be distributed along longitudinal bands or channels, substantially parallel to each other and separated by areas which are substantially free of superabsorbent particles 141.

In an alternative embodiment the particle depositing unit 140 is configured to deposit the superabsorbent particles 141 onto and into the substrate layer 111 in a non-uniform way such that an intermittent distribution of the superabsorbent particles 141 in the longitudinal direction is achieved at a predetermined pitch, and such that transverse areas of superabsorbent particles 141 are formed in the substrate layer 111 which are spaced apart by transverse areas of the substrate layer 111 without superabsorbent particles 141. In the embodiment of figure 5, the apparatus 100 further comprises top layer supplying means 150 configured to provide an essentially endless top layer 151 of non-woven material such that the top layer 151 is positioned on top of the substrate layer 111. The apparatus 100 also comprises an attachment unit configured 160a, 160b to attach said substrate layer 111 to said top layer 151, enclosing the superabsorbent particles 141 therebetween. In a further embodiment, the apparatus further comprises and adhesive providing unit (not shown) configured to provide an adhesive to the substrate layer 111, thereby enabling an easier attachment to a top layer 151. Moreover, in a preferred embodiment the adhesive providing unit is configured to spray glue onto the substrate layer before and/or after superabsorbent particles 141 are deposited on said substrate layer 111. Depending on when the glue is applied onto the substrate layer 111, different suitable glues may be used.

The absorbent structure comprising the substrate layer 111 and the superabsorbent particles 141, and in this particular embodiment also comprising the top layer 151, is manufactured in the form of a continuous tape elongated in a longitudinal direction. It is clear to the skilled person that the resulting continuous absorbent structure can be cut transversely into a plurality of discrete absorbent structures, each of which forms an absorbent structure or at least a part of an absorbent structure of a disposable absorbent article. The resulting discrete absorbent structures are intended to be enclosed between a permeable topsheet and an impermeable backsheet of an absorbent article.

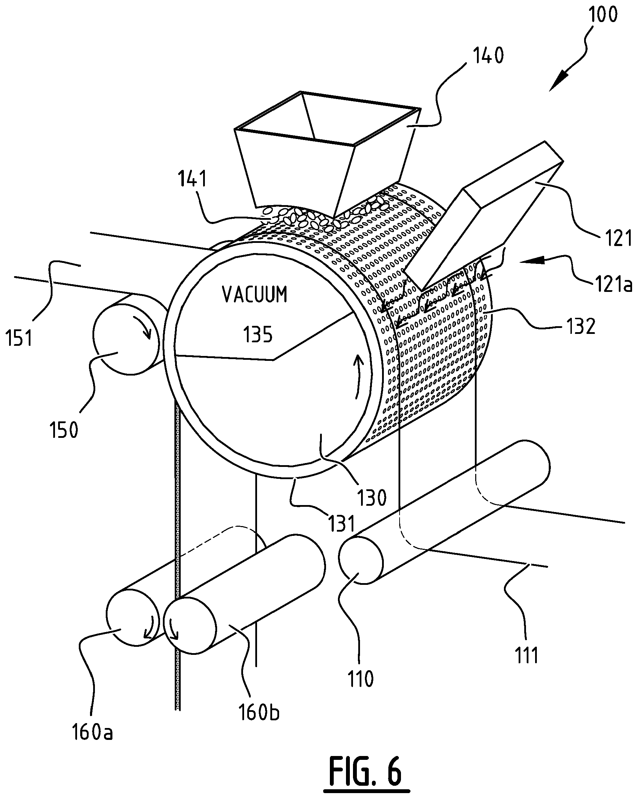

Figure 6 shows a perspective view of an alternative embodiment of the apparatus 100 for manufacturing an absorbent structure. The apparatus 100 comprises substrate layer supplying means 110 which are configured to provide an essentially endless substrate layer 111 of fibrous non-woven material which is heat sensitive. In the illustrated embodiment, the substrate layer supplying means is a cylindrical reel 110 configured to feed a continuous tape or endless layer of substrate material towards an outer surface 131 of a transport device 130. The transport device 130 is illustrated as a rotating member 130 and more in particular as a cylindrical drum rotating around its own central axis and having an outer surface 131 which is provided with perforations. The apparatus 100 comprises a first heat application unit 121 configured to apply heat to said substrate layer 111. In the illustrated embodiment the first heat application unit comprises a first hot air device 121, positioned upstream of a particle depositing unit 140. The first hot air device is configured to generate and direct hot air 121a towards the substrate layer 111. By directing hot air 121a towards the heat sensitive substrate layer 111, fibres within the substrate layer 111 will curl under the influence of the hot air 121a heat which causes the substrate layer 111 to open up. In this manner, an available volume for accommodating superabsorbent particles 141 within the substrate layer 111 is increased, without damaging the fibers of the substrate layer 111 and without negatively affecting the wet and/or dry integrity of the absorbent structure. Furthermore, by opening up the fibrous substrate layer 111 by applying hot air 121a, a deep penetration of superabsorbent particles 141 within the substrate layer may be achieved, and an increased density of superabsorbent particles 141 being distributed within the substrate layer 111 is made possible. The apparatus 100 further comprises the particle depositing unit 140 which is configured to deposit superabsorbent particles 141 onto said substrate layer 111. Because the substrate layer 111 is heated before superabsorbent particles 141 are provided, the fibres of the substrate layer 111 will curl, open up and increase an amount of available volume within the substrate layer 111 to accommodate the superabsorbent particles 141 which are to be provided by the particle depositing unit 140. The apparatus 100 further comprises a sucking unit 135 configured to suck the superabsorbent particles 141 into the substrate layer 111 after the superabsorbent particles 141 are deposited on the substrate layer 111. In the illustrated embodiment, the sucking unit 135 comprises a vacuum chamber 135 within the rotating cylindrical drum 130, which in combination with the perforations 132 in the outer surface 131 of the rotating drum 130 will cause the deposited superabsorbent particles 141 to be sucked deeper into the substrate layer 111. In addition the created vacuum 135 may function to hold the substrate layer 111 onto the outer surface 131 of the rotating drum 130. It is clear to the skilled person that alternative embodiment exist wherein a conveyor belt is used in stead of a rotating drum, and wherein a vacuum chamber 135 is provided below the belt. According to an embodiment, the vacuum chamber 135 has an extension that covers at least the portion of the outer surface 131 between the deposit area where the superabsorbent particles 141 are deposited by means of the particle deposit unit 140, and the area in which the formation of the absorbent structure, or in the case of an additional layer being added, the formation of the absorbent sandwich structure is completed. In a further embodiment, the vacuum chamber 135 may be divided into sectors with different degrees of vacuum, in order to vary the suction action at different areas of the transport device. According to an embodiment it may be beneficial to have greater suction action in the area underneath the particle depositing unit 140.

Figure 7 illustrates an embodiment of a method and apparatus 100 for manufacturing an absorbent structure according to the invention. The apparatus 100 is similar to the apparatus as illustrated in figure 6, with the difference that the apparatus according to figure 7 comprises closed regions 133, 134, 133', 134' on the surface 131 of the rotating member 130, and that two different vacuum chambers (VACUUM 1 and VACUUM 2) are provided. The closed regions 133, 134, 133', 134' provide the functionality of non-suction zones, since the provided vacuum chambers can only exert a suction force through the perforation 132 in the surface 131 of the rotating member 130. This has the effect that substantially no superabsorbent particles will be positioned above the closed regions133, 134, 133', 134' after the substrate layer 111 has passed underneath the particle depositing unit 140. The closed regions 133, 134, 133', 134' comprise at least a first elongate zone 133, 133' and a second elongate zone 134, 134' extending in a circumferential direction of the rotating member 130. Superabsorbent particles 141 are applied via a hopper 140 on the substrate layer 111 on the rotating member 130 such that the regions of the substrate layer 111 located above the perforation 132 are covered with superabsorbent particles and that substantially no superabsorbent particles are present on regions of the substrate layer 111 which are positioned above the closed regions 133, 134, 133', 134'. This way, longitudinal regions or channels 112, 113, 112', 113' which comprise substantially no superabsorbent particles are created within the absorbent structure. In a further step a top layer sheet material 151 is applied on top of the superabsorbent particles on the substrate layer 111, e.g. using a further rotating member 150. In an embodiment, one of said substrate layer and top layer is a top core wrap sheet material, and the other one is a back core wrap sheet material. In a following step the substrate layer 111 is attached to the top layer 151, e.g. in the areas where substantially no superabsorbent material is present, and such that at least a first and a second channel 112, 113 are formed in said absorbent structure. The attaching may be done by applying pressure and/or heat on the substrate layer 111 and/or on the top layer 151, e.g. in the areas where substantially no superabsorbent material is present. This can for example be done. by a rotating member 160a and/or opposite rotating member 160b. Optionally, at least one rotating member 160a, 160b is provided with at least a first and a second seal rib (not shown) dimensioned for applying pressure and heat on the substrate layer 111 in the areas where substantially no superabsorbent material is present in order to create the first and second channel 112, 113, respectively. In addition to or alternative to the use of closed regions to create areas wherein substantially no superabsorbent particles 141 are present the hot air device 121 may be configured to direct hot air towards multiple surfaces of the substrate layer 111, wherein the multiple surface are separated by a surface on which substantially no superabsorbent particles are to be deposited. To this end, the hot air device 121 may be equipped with a plurality of precision nozzles (not shown) wherein each precision nozzle is configured to direct hot air to a surface from among the multiple surfaces on the substrate layer 111. In this manner, areas where substantially no superabsorbent material is present can be created in the substrate layer 111 and in the resulting absorbent structure. Alternatively or in addition, areas where substantially no superabsorbent material is present can be created in the substrate layer and in the resulting absorbent structure by depositing the superabsorbent particles 141 onto and into the substrate layer in 111 a controlled manner such that substantially no superabsorbent particles are deposited on the areas which need to be substantially free of superabsorbent particles, e.g. by using precision hoppers, by applying vacuum, and/or by using closed regions on the surface 131 of the rotating member 130.

Figure 8 illustrate an exemplary embodiment of an absorbent article, here a diaper, comprising an absorbent core which is manufactured with an apparatus as described in view of figure 7. Although in figure 7, the closed regions 133, 134, 133', 134' and resulting channels 112, 113, 112', 113' have been illustrated and described as comprising the shape of two elongate rectangles being substantially parallel to each other, it is clear to the skilled person that numerous alternative embodiments exist, wherein the at least two channels 112, 113, 112', 113' have other shapes and configurations as compared to those as shown in figure 7. An exemplary embodiment of an absorbent article comprising an absorbent structure with total of four channels 140, 150, 160, 170 is illustrated in figure 8. The skilled person understands that the absorbent article may also be a pant or an adult incontinence garment or the like. Absorbent article 100 is provided with a first and second channel 140, 150, each extending from a crotch region CR in the direction of transverse edge of the absorbent core. First channel 140 and second channel 150 are substantially parallel and run in the longitudinal direction of absorbent core. However, it is also possible for first and second channel 140, 150 to extend under a small angle with respect to the longitudinal direction of absorbent core, e.g. an angle between 5 and 10°.

The illustrated absorbent article 100 is further provided with a third and a fourth channel 160, 170 located at a distance d34 of each other. Third and fourth channel 160, 170 each extend from crotch region CR in the direction of a second transverse edge. In the illustrated embodiment, the distance d12 between first and second channel 140, 150 is different from the distance d34 between third and fourth channel 160, 170. Third channel 160 and fourth channel 170 are substantially parallel and run in the longitudinal direction of absorbent core 130. However, it is also possible for third and fourth channel 160, 170 to extend under a small angle with respect to the longitudinal direction of absorbent core 130, e.g. an angle between 5 and 10°. For example, third and fourth channel 160, 170 may be diverging slightly outwardly in the direction of a transverse edge of the absorbent core. Preferably third channel 160 and fourth channel 170 are arranged symmetrically with respect to a longitudinal center line CL of absorbent core.

Outside of the plurality of channels 140, 150, 160, 170, absorbent core 130 has a maximum thickness. Preferably, each channel 140, 150, 160, 170 extends through at least 90 % of the maximum thickness of the absorbent core, more preferably through 100% of the thickness of absorbent core, such that, in the channel 140, 150, 160, 170, substantially no absorbent material is present.

Channels 140, 150, 160, 170 guide urine U or any other aqueous liquid through the side walls of channels 140, 150, 160, 170 into the absorbent core. Those side walls create an additional path along which the liquid can flow into the absorbent core and enhance the diffusion of the liquid into the absorbent core. Also, because of the swelling of the core material of absorbent core, the outer bands of the absorbent core will rotate around channels 140, 150, 160, 170 . In that manner the diaper takes the shape of a tub or cup, such that any liquid which would not yet be absorbed by the absorbent material is maintained in the tub shape. This results in a better protection against leakage and a diaper fitting perfectly to the body. Hence the diaper of figure 8 will create more freedom of movement for the wearer of a wetted diaper.

It is clear to the skilled person that channels may be provided by means of continuous attachments in the transversal direction of the absorbent core and/or continuous attachments in the longitudinal direction of the absorbent core and/or discontinuous attachments in the transversal direction of the absorbent core and/or discontinuous attachments in the longitudinal direction of the absorbent core.

The chassis of the diaper 100 in figure 8 preferably further includes side panels or ears 210, elasticized leg cuffs 230 and elastic waist elements (not shown). A front end portion of diaper 100 is configured as a front waist region 100a. The opposite rear end portion is configured as a back waist region 100b of diaper 100. An intermediate portion of diaper 100 is configured as crotch region CR, which extends longitudinally between first and second waist regions 100a and 100b. Waist regions 100a and 100b may include elastic waist elements such that they gather about the waist of the wearer to provide improved fit and containment. Crotch region CR is that portion of diaper 100 which, when the diaper 100 is worn, is generally positioned between the wearer's legs. The periphery of diaper 100 is defined by the outer edges of the diaper 100 in which longitudinal edges run generally parallel to a longitudinal axis of diaper 100 and transverse end edges run between the longitudinal edges generally parallel to a transverse axis of diaper 100. The chassis also comprises a fastening system, which may include at least one fastening or securing member 212 and at least one landing zone 220. The various components within diaper 100 may be bound, joined or secured by any method known in the art, for example by adhesives in uniform continuous layers, patterned layers or arrays of separate lines, spirals or spots. Top core wrap sheet, topsheet, back core wrap sheet, backsheet, absorbent material and other components may be assembled in a variety of well-known configurations and are well known in the art.

The absorbent core may comprise any absorbent material that is generally compressible, conformable, non-irritating to the wearer's skin, and capable of absorbing and retaining bodily exudates. The absorbent core may comprise a wide variety of liquid absorbent materials commonly used in absorbent articles. Preferably, the absorbent core is substantially fluffless and comprises superabsorbent polymers. Also, the absorbent core may comprise a combination of cellulosic fluff pulp and superabsorbent polymers. The absorbent core may be configured to extend substantially the full length and/or width of diaper 100. However, as in the embodiment of figure 8, preferably the absorbent structure is not coextensive with the entire diaper 100 and is limited to certain regions of diaper 100 including crotch region CR. In various embodiments, the absorbent core extends to the edges of diaper 100 but the absorbent material is concentrated in the crotch region CR or another target zone of the diaper 100. In figure 8, the absorbent core is shown as having a substantially rectangular configuration, however, the absorbent core may be shaped differently, such as, elliptical, dogbane shaped, T-shaped or I-shaped. More in particular the width of the front portion may be smaller than the width of the rear portion of the absorbent core.

Examples of commonly occurring absorbent materials used for the absorbent core are cellulosic fluff pulp, tissue layers, highly absorbent polymers (so called superabsorbents), absorbent foam materials, absorbent nonwoven materials or the like. It is common to combine cellulosic fluff pulp with superabsorbent polymers in an absorbent core. Superabsorbent polymers are water-swellable, water-insoluble organic or inorganic materials capable of absorbing at least about 20 times its weight and in an aqueous solution containing 0.9 weight percent of sodium chloride.

Diaper 100 may also utilize a pair of containment walls or cuffs 230. Each cuff 230 is a longitudinally extending wall structure preferably positioned on each side of the absorbent core and spaced laterally from the center line CL. Preferably, cuffs 230 are attached, for example, by adhesive or sonic bonding to the lower structure. Preferably, cuffs 230 are equipped with elastic members. When released or otherwise allowed relaxing, the elastic members retract inwardly. When diaper 100 is worn, the elastic members function to contract cuffs 230 about the buttocks and the thighs of the wearer in a manner, which forms a seal between diaper 100, the buttocks and the thighs.

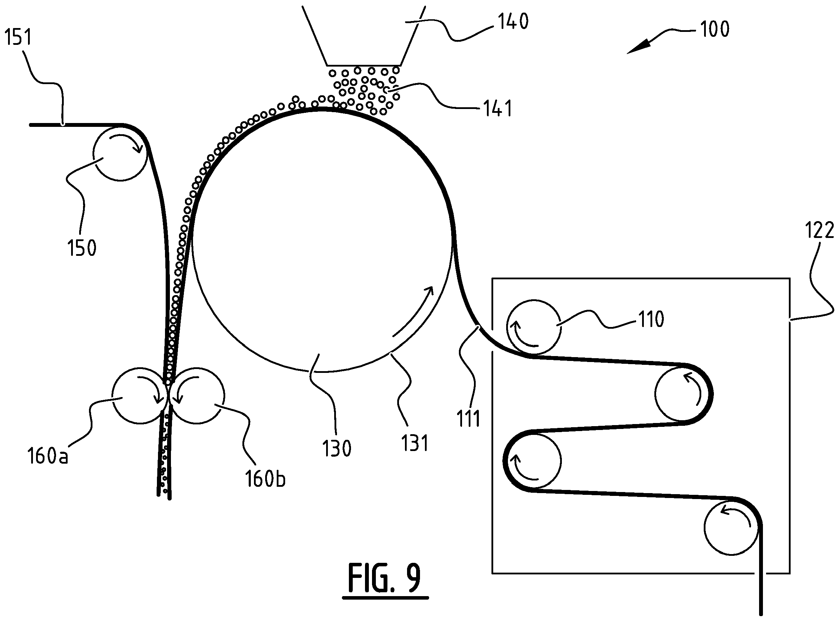

The waist regions 100a and 100b each comprise a central region and a pair of side panels or ears 210, 210' which typically comprise the outer lateral portions of the waist regions. These side panels 210, 210' may be unitary with the chassis or may be attached or joined thereto by any means know in the art. Preferably, the side panels 210 positioned in the back waist region 100b are flexible, extensible and/or elastic in at least the lateral direction. In another embodiment the side panels 210 are non-elastic, semi-rigid, rigid and/or stiff. In order to keep diaper 100 in place about the wearer, preferably at least a portion of the back waist region 100b is attached by fastening or securing members 212 to at least a portion of the front waist region 100a. The fastening or securing members 212 may be e.g. adhesive, mechanical fasteners, hook and loop features, conceivable strings and/or combinations thereof. The fastening or securing members 212 may also be co-adhesive such that they adhere to each other but not other materials. Preferably the materials making up the fastening or securing members 212 are flexible, extensible and/or elastic, allowing them to better conform to the shape and movements of the body and thus, to reduce the likelihood that the fastening system will irritate or injure the wearer's skin. Alternatively, the absorbent article may be pants and the like. In this configuration, the absorbent article may or may not have fastening members. Figure 9 illustrates an embodiment of the apparatus for manufacturing an absorbent structure which is similar to the apparatus as shown in figure 5, with the difference that in stead of a first hot air device 121 which is configured to direct hot air 121a towards the substrate layer 111, the apparatus 100 in figure 7 comprises a heating chamber 122 which is positioned upstream of the particle depositing unit 140 and is configured to guide the substrate layer 111 therethrough. By guiding the substrate layer 111 through a heating chamber 122 the fibers of the substrate layer 111 will heat up and consequently curl and open up, thereby allowing an increased volume within the substrate layer 111 to be available for receiving and accommodating superabsorbent particles 141. In an alternative embodiment an additional hot air device is provided between the heating chamber 122 and the particle depositing unit 140. In yet another embodiment, the heating chamber 122 is provided between a hot air device and the particle depositing unit 140.

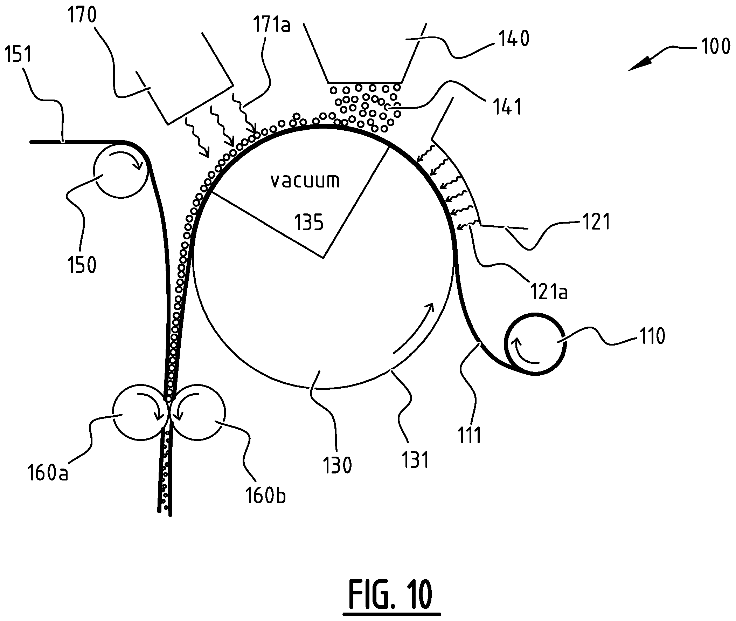

Figure 10 illustrates an embodiment of the apparatus for manufacturing an absorbent structure which is similar to the apparatus as shown in figure 5, with the difference that the apparatus 100 of figure 10 comprises a second heat application unit 170 positioned downstream of the particle depositing unit 140 which is configured to apply heat 171a to said substrate layer 111 after superabsorbent particles 141 have been deposited thereon. In the embodiment of figure 10, the second heat application unit 170 is a second hot air device to generate and direct hot air 171a towards the substrate layer 111 comprising the superabsorbent particles 141. In an embodiment, the second heat application unit 170 comprises a heating chamber alternative, or in addition to the second hot air device 170. The apparatus 100 of figure 10 further comprises a sucking unit 135 configured to suck the superabsorbent particles 141 deeper into the substrate layer 111 after the superabsorbent particles 141 are deposited on the substrate layer 111. In the illustrated embodiment, the sucking unit 135 comprises a vacuum chamber 135 within the rotating wheel 130. The outer surface 131 of the rotating wheel 130 is provided with perforations (not shown). In combination with the perforations 132 in the outer surface 131 of the rotating element 130, the vacuum chamber will cause the deposited superabsorbent particles 141 to be sucked deeper into the substrate layer 111.

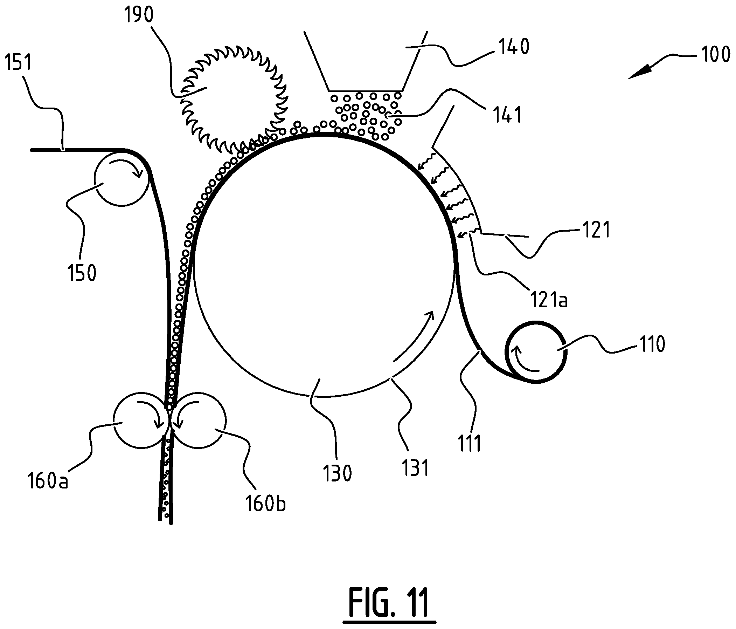

Figure 11 illustrates an embodiment of the apparatus for manufacturing an absorbent structure which is similar to the apparatus as shown in figure 10, with the difference that the apparatus 100 of figure 11 comprises a combing unit 190 in stead of a heat application unit. The combing unit 190 is positioned downstream of the particle depositing unit 140 and is configured to comb through the substrate layer 111. In addition, or alternative to the combing unit 190 being positioned downstream of the particle depositing unit 140, a combing unit may be provided upstream of the particle depositing unit 140. The combing unit comprises a cylinder with a toothed portion having protruding elements configured to comb through the substrate layer 111. In combination with one, two or more provided heat application units 120, 121, 122 the combing unit may produce an increase in the volume of the substrate layer 111 which is available for receiving and/or accommodating superabsorbent particles 141. Such an increase in the available volume of the substrate layer 111, preferably in combination with the suction action of the vacuum chamber 135 (not shown in figure 11) produces a deep penetration of the superabsorbent particles 141 within the substrate layer 111 and a substantial increase in the density of superabsorbent particles 141 which can be accommodated in substrate layer 111.

## Claims

1. A method of manufacturing an absorbent structure comprising a substrate layer and superabsorbent particles, said method comprising the steps of:
- providing an essentially endless substrate layer (111) of fibrous non-woven material which is heat sensitive, wherein the non-woven material comprises binder fibres consisting of sheath-core bicomponent fibres, and carrier fibres consisting of polyester fibres;
- applying heat to said substrate layer (111), wherein the substrate layer (111) is heated to a temperature of between 80°C and 120°C such that an available volume for accommodating superabsorbent particles (141) within the substrate layer (111) is increased; and
- depositing superabsorbent particles (141) on said substrate layer (111).

2. The method according to claim 1, wherein applying heat to the substrate layer (111) is done by directing hot air (121a) towards the substrate layer (111), while the substrate layer (111) is guided along a rotating member (130).

3. The method according to claim 2, wherein the hot air (121a) has a temperature between 80°C and 180°C, more preferably between 100°C and 160°C and most preferably between 120°C and 150°C.

4. The method according to claim 2 or 3, wherein the hot air is directed towards the substrate layer from a distance of 1 to 20 cm, preferably 2 to 15cm, more preferably 3 to 12 cm, and most preferably 5 to 10cm from the substrate layer.

5. The method according to any one of the preceding claims 2-4, wherein the substrate layer is guided along the rotating member at a speed of at least 1 m/s, preferably at least 2 m/s, more preferably at least 3 m/s, and most preferably at least 3,5 m/s.

6. The method according to any one of the preceding claims 2-5, wherein the hot air is directed towards a surface of the substrate layer having a width of at least 50%, preferably at least 60%, more preferably at least 70%, even more preferably 80% and most preferably at least 90% of the width of the substrate layer.

7. The method according to any one of the preceding claims, wherein applying heat to the substrate layer (111) is done by guiding the substrate layer (111) through a heating chamber (122).

8. The method according to claim 7, wherein the temperature in the heating chamber is between 80°C and 120°C.

9. The method according to claim 7 or 8, wherein the substrate layer is guided through the heating chamber at a speed of at least 1 m/s, preferably at least 2 m/s, more preferably at least 3 m/s, and most preferably at least 3,5 m/s.

10. The method according to any one of the preceding claims 7-9, wherein the substrate layer is guided through the heating chamber for a distance of 1 to 30m, preferably 2 to 25m, more preferably 3 to 20m, and more preferably 5 to 15m.

11. The method according to any one of the preceding claims, further comprising the step of applying heat to said substrate layer (111) after superabsorbent particles (141) have been deposited thereon.

12. The method according to any one of the preceding claims, further comprising the step of combing the substrate layer (111) before superabsorbent particles (141) are deposited thereon.

13. The method according to any one of the preceding claims, further comprising the step of combing the substrate layer (111) after superabsorbent particles have been deposited thereon.

14. The method according to any one of the preceding claims, further comprising the steps of:
- providing an essentially endless top layer (151);
- applying the top layer (151) on top of the substrate layer (111); and
- attaching said substrate layer (111) to said top layer (151), thereby enclosing the superabsorbent particles (141) therebetween.

15. An apparatus (100) for manufacturing an absorbent structure comprising a substrate layer and superabsorbent particles, said apparatus comprising:
- substrate layer supplying means (110) configured to provide an essentially endless substrate layer (111) of fibrous non-woven material which is heat sensitive, wherein the non-woven material comprises binder fibres consisting of sheath-core bicomponent fibres, and carrier fibres consisting of polyester fibres;
- a first heat application unit (120, 121,122) configured to apply heat to said substrate layer (111), wherein the substrate layer (111) is heated to a temperature of between 80°C and 120°C such that an available volume for accommodating superabsorbent particles (141) within the substrate layer (111) is increased; and
- a particle depositing unit (140) configured to deposit superabsorbent particles (141) on said substrate layer (111).

## Patentansprüche

1. Verfahren zum Herstellen einer absorbierenden Struktur, umfassend eine Substratschicht und superabsorbierende Partikel, wobei das Verfahren die Schritte umfasst:
- Bereitstellen einer im Wesentlichen endlosen Substratschicht (111) aus faserigem Vliesmaterial, die wärmeempfindlich ist, wobei das Vliesmaterial Bindemittelfasern, die aus Mantelkern-Bikomponentenfasern bestehen, und Trägerfasern umfasst, die aus Polyesterfasern bestehen;
- Aufbringen von Wärme auf die Substratschicht (111), wobei die Substratschicht (111) auf eine Temperatur zwischen 80 °C und 120 °C erhitzt wird, sodass ein verfügbares Volumen zur Aufnahme von superabsorbierenden Partikeln (141) innerhalb der Substratschicht (111) erhöht wird; und
- Abscheiden von superabsorbierenden Partikeln (141) auf die Substratschicht (111).

2. Verfahren nach Anspruch 1, wobei das Aufbringen von Wärme auf die Substratschicht (111) durch Richten von heißer Luft (121a) zur Substratschicht (111) erfolgt, während die Substratschicht (111) entlang eines Rotationselements (130) geleitet wird.

3. Verfahren nach Anspruch 2, wobei die heiße Luft (121a) eine Temperatur zwischen 80 °C und 180 °C, mehr bevorzugt zwischen 100 °C und 160 °C und am meisten bevorzugt zwischen 120 °C und 150 °C aufweist.

4. Verfahren nach Anspruch 2 oder 3, wobei die heiße Luft aus einem Abstand von 1 bis 20 cm, vorzugsweise 2 bis 15 cm, mehr bevorzugt 3 bis 12 cm und am meisten bevorzugt 5 bis 10 cm von der Substratschicht zur Substratschicht gerichtet wird.

5. Verfahren nach einem der vorstehenden Ansprüche 2 bis 4, wobei die Substratschicht entlang des Rotationselements mit einer Geschwindigkeit von mindestens 1 m/s, vorzugsweise mindestens 2 m/s, mehr bevorzugt mindestens 3 m/s und am meisten bevorzugt mindestens 3,5 m/s geleitet wird.

6. Verfahren nach einem der vorstehenden Ansprüche 2 bis 5, wobei die heiße Luft zu einer Oberfläche der Substratschicht gerichtet wird, die eine Breite von mindestens 50 %, vorzugsweise mindestens 60 %, mehr bevorzugt mindestens 70 %, noch mehr bevorzugt 80 % und am meisten bevorzugt mindestens 90 % der Breite der Substratschicht aufweist.

7. Verfahren nach einem der vorstehenden Ansprüche, wobei das Aufbringen von Wärme auf die Substratschicht (111) durch Leiten der Substratschicht (111) durch eine Heizkammer (122) erfolgt.

8. Verfahren nach Anspruch 7, wobei die Temperatur in der Heizkammer zwischen 80 °C und 120 °C liegt.

9. Verfahren nach Anspruch 7 oder 8, wobei die Substratschicht mit einer Geschwindigkeit von mindestens 1 m/s, vorzugsweise mindestens 2 m/s, mehr bevorzugt mindestens 3 m/s und am meisten bevorzugt mindestens 3,5 m/s durch die Heizkammer geleitet wird.

10. Verfahren nach einem der vorstehenden Ansprüche 7 bis 9, wobei die Substratschicht für einen Abstand von 1 bis 30 m, bevorzugt 2 bis 25 m, mehr bevorzugt 3 bis 20 m und mehr bevorzugt 5 bis 15 m durch die Heizkammer geleitet wird.

11. Verfahren nach einem der vorstehenden Ansprüche, ferner umfassend den Schritt des Aufbringens von Wärme auf die Substratschicht (111), nachdem superabsorbierende Partikel (141) darauf abgeschieden worden sind.

12. Verfahren nach einem der vorstehenden Ansprüche, ferner umfassend den Schritt eines Kämmens der Substratschicht (111) vor dem Abscheiden von superabsorbierenden Partikeln (141).

13. Verfahren nach einem der vorstehenden Ansprüche, ferner umfassend den Schritt des Kämmens der Substratschicht (111), nachdem superabsorbierende Partikel darauf abgeschieden worden sind.

14. Verfahren nach einem der vorstehenden Ansprüche, ferner umfassend die Schritte:
- Bereitstellen einer im Wesentlichen endlosen oberen Schicht (151);
- Aufbringen der oberen Schicht (151) auf die Substratschicht (111); und
- Anbringen der Substratschicht (111) an die obere Schicht (151), wodurch die superabsorbierenden Partikel (141) dazwischen eingeschlossen werden.

15. Vorrichtung (100) zum Herstellen einer absorbierenden Struktur, umfassend eine Substratschicht und superabsorbierende Partikel umfasst, wobei die Vorrichtung umfasst:
- Substratschichtzuführmittel (110), die konfiguriert sind, um eine im Wesentlichen endlose Substratschicht (111) aus faserigem Vliesmaterial bereitzustellen, die wärmeempfindlich ist, wobei das Vliesmaterial Bindemittelfasern, die aus Mantelkern-Bikomponentenfasern bestehen, und Trägerfasern umfasst, die aus Polyesterfasern bestehen;
- eine erste Wärmeaufbringungseinheit (120, 121,122), die konfiguriert ist, um Wärme auf die Substratschicht (111) aufzubringen, wobei die Substratschicht (111) auf eine Temperatur zwischen 80 °C und 120 °C erhitzt wird, sodass ein verfügbares Volumen zur Aufnahme von superabsorbierenden Partikeln (141) innerhalb der Substratschicht (111) erhöht wird; und
- eine Partikelabscheideeinheit (140), die konfiguriert ist, um superabsorbierende Partikel (141) auf der Substratschicht (111) abzuscheiden.

## Revendications

1. Procédé de fabrication d'une structure absorbante comprenant une couche de substrat et des particules très absorbantes, ledit procédé comprenant les étapes de :
fourniture d'une couche de substrat (111) sensiblement continue de matériau fibreux non tissé qui est sensible à la chaleur, dans lequel le matériau non tissé comprend des fibres liantes constituées par des fibres à gaine-âme bi-composants et des fibres porteuses constituées par des fibres de polyester ;
application de chaleur sur ladite couche de substrat (111), dans lequel la couche de substrat (111) est chauffée à une température comprise entre 80°C et 120°C de telle sorte qu'un volume de réception des particules très absorbantes (141) disponible à l'intérieur de la couche de substrat (111) est augmenté ; et
dépôt de particules très absorbantes (141) sur ladite couche de substrat (111).

2. Procédé selon la revendication 1, dans lequel l'application de chaleur sur la couche de substrat (111) est réalisée en dirigeant de l'air chaud (121a) vers la couche de substrat (111), pendant que la couche de substrat (111) est guidée le long d'un élément tournant (130).

3. Procédé selon la revendication 2, dans lequel l'air chaud (121a) présente une température comprise entre 80°C et 180°C, plus préférablement, entre 100°C et 160°C et le plus préférablement, entre 120°C et 150°C.

4. Procédé selon la revendication des 2 ou 3, dans lequel l'air chaud est dirigé vers la couche de substrat à partir d'une distance de 1 à 20 cm, de préférence, de 2 à 15 cm, plus préférablement, de 3 à 12 cm et le plus préférablement, de 5 à 10 cm par rapport à la couche de substrat.

5. Procédé selon l'une quelconque des revendications 2 à 4 précédentes, dans lequel la couche de substrat est guidée le long de l'élément tournant à une vitesse supérieure ou égale à 1 m/s, de préférence, supérieure ou égale à 2 m/s, plus préférablement, supérieure ou égale à 3 m/s, et le plus préférablement, supérieure ou égale à 3,5 m/s.

6. Procédé selon l'une quelconque des revendications 2 à 5 précédentes, dans lequel l'air chaud est dirigé vers une surface de la couche de substrat présentant une largeur supérieure ou égale à 50%, de préférence, supérieure ou égale à 60%, plus préférablement, supérieure ou égale à 70%, encore plus préférablement, de 80% et le plus préférablement, supérieure ou égale à 90% de la largeur de la couche de substrat.

7. Procédé selon l'une quelconque des revendications précédentes, dans lequel l'application de chaleur sur la couche de substrat (111) est réalisée en guidant la couche de substrat (111) à travers un compartiment de chauffage (122).

8. Procédé selon la revendication 7, dans lequel la température dans le compartiment de chauffage est comprise entre 80°C et 120°C.

9. Procédé selon la revendication 7 ou 8, dans lequel la couche de substrat est guidée à travers le compartiment de chauffage à une vitesse supérieure ou égale à 1 m/s, de préférence, supérieure ou égale à 2 m/s, plus préférablement, supérieure ou égale à 3 m/s et le plus préférablement, supérieure ou égale à 3,5 m/s.

10. Procédé selon l'une quelconque des revendications 7 à 9 précédentes, dans lequel la couche de substrat est guidée à travers le compartiment de chauffage sur une distance de 1 à 30 m, de préférence, de 2 à 25 m, plus préférablement, de 3 à 20 m, et le plus préférablement, de 5 à 15 m.

11. Procédé selon l'une quelconque des revendications précédentes, comprenant, en outre, l'étape d'application de chaleur sur ladite couche de substrat (111) après que des particules très absorbantes (141) ont été déposées dessus.

12. Procédé selon l'une quelconque des revendications précédentes, comprenant, en outre, l'étape de peignage de la couche de substrat (111) avant que des particules très absorbantes (141) soient déposées dessus.

13. Procédé selon l'une quelconque des revendications précédentes, comprenant, en outre, l'étape de peignage de la couche de substrat (111) après que des particules très absorbantes ont été déposées dessus.

14. Procédé selon l'une quelconque des revendications précédentes, comprenant, en outre, les étapes de :
fourniture d'une couche supérieure (151) sensiblement continue ;
application de la couche supérieure (151) au-dessus de la couche de substrat (111) ; et
fixation de ladite couche de substrat (111) sur ladite couche supérieure (151), enfermant ainsi les particules très absorbantes (141) entre les deux.

15. Dispositif (100) destiné à fabriquer une structure absorbante comprenant une couche de substrat et des particules très absorbantes, ledit dispositif comprenant :
un moyen d'alimentation de couche de substrat (110) configuré de manière à délivrer une couche de substrat (111) sensiblement continue de matériau fibreux non tissé qui est sensible à la chaleur, dans lequel le matériau non tissé comprend des fibres liantes constituées par des fibres à gaine-âme bi-composants et des fibres porteuses constituées par des fibres de polyester ;
une première unité d'application de chaleur (120, 121, 122) configurée de manière à appliquer de la chaleur sur ladite couche de substrat (111), dans laquelle la couche de substrat (111) est chauffée à une température comprise entre 80°C et 120°C de telle sorte qu'un volume disponible afin de recevoir des particules très absorbantes (141) à l'intérieur de la couche de substrat (111) est augmenté ; et
une unité de dépôt de particules (140) configurée de manière à déposer des particules très absorbantes (141) sur ladite couche de substrat (111).
